# EUROPEAN PATENT SPECIFICATION

(11) **EP 0 702 721 B1**
(45) Date of publication and mention of the grant of the patent: **03.01.2001**
(21) Application number: 94920443.2
(22) Date of filing: 09.06.1994
(51) Int. Cl.: C12N 15/62, C12N 15/80

(54) **PROCESS FOR PRODUCING FUSION PROTEINS COMPRISING SCFV FRAGMENTS BY A TRANSFORMED MOULD**
VERFAHREN ZUR HERSTELLUNG VON FUSIONSPROTEINEN, DIE SCFV FRAGMENTE ENTHALTEN, IN TRANSFORMIERTEN SCHIMMELPILZEN
PROCEDE DE PRODUCTION DE PROTEINES DE FUSION COMPRENANT DES FRAGMENTS DE SCFV A L'AIDE D'UN MOULE TRANSFORME

(30) Priority: 14.06.1993 EP 93201706; 09.06.1993 EP 93201661; 09.06.1993 EP 93201660
(43) Date of publication of application: 27.03.1996
(73) Proprietor: UNILEVER N.V., 3013 AL Rotterdam (NL); UNILEVER PLC, London EC4P 4BQ (GB); NEDERLANDSE ORGANISATIE VOOR TOEGEPAST-NATUURWETENSCHAPPELIJK ONDERZOEK TNO, 2628 VK Delft (NL)
(72) Inventor: FRENKEN, Leon Gerardus Joseph, NL-3011 MP Rotterdam (NL); VAN GORCOM, Robert F.M., NL-2622 DE Delft (NL); HESSING, Johanna G.M., NL-2624 PG Delft (NL); VAN DEN HONDEL, Cornelis Antonius M.J.J., NL-2804 PZ Gouda (NL); MUSTERS, Wouter, NL-3141 RD Maassluis (NL); VERBAKEL, Johannes MariaA., NL-3155 GC Maasland (NL); VERRIPS, Cornelis Theodorus, NL-3142 KB Maassluis (NL)
(74) Representative: Smulders, Theodorus A.H.J., Ir.
(86) International application number: EP9401906
(87) International publication number: WO9429457

(56) References cited:
- EP-A- 0 614 982
- WO-A-92/01797
- WO-A-93/08300
- JOURNAL OF BIOLOGICAL CHEMISTRY., vol.266, no.25, 5 September 1991, BALTIMORE US pages 16343 - 16349 LAROCHE ET AL. 'Characterization of a recombinant single-chain molecule comprising the variable domains of a monoclonal antibody specific for human fibrin fragment D-dimer'
- TRENDS IN BIOTECHNOLOGY, vol.9, no.4, April 1991, CAMBRIDGE GB pages 132 - 137 BIRD AND WALKER 'Single chain antibody variable regions'
- JOURNAL OF BIOLOGICAL CHEMISTRY., vol.266, no.32, 15 November 1991, BALTIMORE US pages 21874 - 21879 ANAND ET AL. 'Bacterial expression and secretion of various single-chain Fv genes encoding proteins specific for a Salmonella serotype B O-antigen'
- BIOTECHNOLOGY, vol.11, no.1, January 1993, NEW YORK US pages 71 - 76 WU ET AL. 'Efficient production of a functional single-chain antidigoxin antibody via an engineered Bacillus subtilis expression-secretion system'
- ANTONIE VAN LEEUWENHOEK, vol.61, no.2, February 1992, DORDRECHT, NL pages 153 - 160 VAN DEN HONDEL ET AL. 'Production of extracellular proteins by the filamentous fungus Aspergillus'

### SEQUENCE LISTING

(1) GENERAL INFORMATION:
   (i) APPLICANT:
      (A) NAME: UNILEVER N.V.
      (B) STREET: Weena 455
      (C) CITY: Rotterdam
      (E) COUNTRY: The Netherlands
      (F) POSTAL CODE (ZIP): NL-3013 AL

      (A) NAME: UNILEVER PLC
      (B) STREET: Unilever House Blackfriars
      (C) CITY: London
      (E) COUNTRY: United Kingdom
      (F) POSTAL CODE (ZIP): EC4P 4BQ

      (A) NAME: NEDERLANDSE ORGANISATIE VOOR TOEGEPAST-NATUURWETENSCHAPPELIJK ONDERZOEK TNO
      (B) STREET: Schoemakersstraat 97
      (C) CITY: Delft
      (E) COUNTRY: The Netherlands
      (F) POSTAL CODE (ZIP): NL-2628 VK

      (A) NAME: Leon Gerardus Joseph FRENKEN
      (B) STREET: Geldersestraat 90
      (C) CITY: Rotterdam
      (E) COUNTRY: The Netherlands
      (F) POSTAL CODE (ZIP): NL-3011 MP

      (A) NAME: Robert F.M. van GORCOM
      (B) STREET: Liberiastraat 7
      (C) CITY: Delft
      (E) COUNTRY: The Netherlands
      (F) POSTAL CODE (ZIP): NL-2622 DE

      (A) NAME: Johanna G.M. HESSING
      (B) STREET: Adema van Scheltemaplein 38
      (C) CITY: Delft
      (E) COUNTRY: The Netherlands
      (F) POSTAL CODE (ZIP): NL-2624 PG

      (A) NAME: Cornelis Antonius M.J.J. van den HONDEL
      (B) STREET: Waterlelie 124
      (C) CITY: Gouda
      (E) COUNTRY: The Netherlands
      (F) POSTAL CODE (ZIP): NL-2804 PZ

      (A) NAME: Wouter MUSTERS
      (B) STREET: Wipperspark 138
      (C) CITY: Maassluis
      (E) COUNTRY: The Netherlands
      (F) POSTAL CODE (ZIP): NL-3141 RD

      (A) NAME: Johannes Maria A. VERBAKEL
      (B) STREET: Ingeland 9
      (C) CITY: Maasland
      (E) COUNTRY: The Netherlands
      (F) POSTAL CODE (ZIP): NL-3155 GC

      (A) NAME: Cornelis Theodorus VERRIPS
      (B) STREET: Hagedoorn 18
      (C) CITY: Maassluis
      (E) COUNTRY: The Netherlands
      (F) POSTAL CODE (ZIP): NL-3142 KB
   (ii) TITLE OF INVENTION:
      Process for producing fusion proteins comprising ScFv fragments by a transformed mould
   (iii) NUMBER OF SEQUENCES: 45
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 1:
   Gly Gly Gly Gly Ser Gly Gly Gly Gly Ser Gly Gly Gly Gly Ser
    1               5                  10                  15
(2) INFORMATION FOR SEQ ID NO: 2:
   (i) SEQUENCE CHARACTERISTICS:
      (A) LENGTH: 895 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..855
      (ix) FEATURE:
         (A) NAME/KEY: CDS
         (B) LOCATION: 1..855
      (xi) SEQUENCE DESCRIPTION: SEQ ID NO: 2:
(2) INFORMATION FOR SEQ ID NO: 3:
   (i) SEQUENCE CHARACTERISTICS:
      (A) LENGTH: 285 amino acids
      (B) TYPE: amino acid
      (D) TOPOLOGY: linear
   (ii) MOLECULE TYPE: protein
   (xi) SEQUENCE DESCRIPTION: SEQ ID NO: 3:
   Lys Leu Ala Cys Lys Phe Tyr Phe Lys Glu Thr Val Ile Met Lys Tyr
    1               5                  10                  15
   Leu Leu Pro Thr Ala Ala Ala Gly Leu Leu Leu Leu Ala Ala Gln Pro
               20                  25                  30
   Ala Met Ala Gln Val Gln Leu Gln Glu Ser Gly Pro Gly Leu Val Ala
           35                  40                  45
   Pro Ser Gln Ser Leu Ser Ile Thr Cys Thr Val Ser Gly Phe Ser Leu
       50                  55                  60
   Thr Gly Tyr Gly Val Asn Trp Val Arg Gln Pro Pro Gly Lys Gly Leu
    65                 70                  75                  80
   Glu Trp Leu Gly Met Ile Trp Gly Asp Gly Asn Thr Asp Tyr Asn Ser
                    85                  90                  95
   Ala Leu Lys Ser Arg Leu Ser Ile Ser Lys Asp Asn Ser Lys Ser Gln
               100                 105                 110
   Val Phe Leu Lys Met Asn Ser Leu His Thr Asp Asp Thr Ala Arg Tyr
           115                 120                 125
   Tyr Cys Ala Arg Glu Arg Asp Tyr Arg Leu Asp Tyr Trp Gly Gln Gly
       130                 135                 140
   Thr Thr Val Thr Val Ser Ser Gly Gly Gly Gly Ser Gly Gly Gly Gly
   145                 150                 155                 160
   Ser Gly Gly Gly Gly Ser Asp Ile Glu Leu Thr Gln Ser Pro Ala Ser
                   165                 170                 175
   Leu Ser Ala Ser Val Gly Glu Thr Val Thr Ile Thr Cys Arg Ala Ser
               180                 185                 190
   Gly Asn Ile His Asn Tyr Leu Ala Trp Tyr Gln Gln Lys Gln Gly Lys
           195                 200                 205
   Ser Pro Gln Leu Leu Val Tyr Tyr Thr Thr Thr Leu Ala Asp Gly Val
       210                 215                 220
   Pro Ser Arg Phe Ser Gly Ser Gly Ser Gly Thr Gln Tyr Ser Leu Lys
   225                 230                 235                 240
   Ile Asn Ser Leu Gln Pro Glu Asp Phe Gly Ser Tyr Tyr Cys Gln His
                   245                 250                 255
   Phe Trp Ser Thr Pro Arg Thr Phe Gly Gly Gly Thr Lys Leu Glu Ile
               260                 265                 270
   Lys Arg Glu Gln Lys Leu Ile Ser Glu Glu Asp Leu Asn
           275                 280                 285
(2) INFORMATION FOR SEQ ID NO: 4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 4:
   TCGAGATCAA ACGGTAATGA G
               21
(2) INFORMATION FOR SEQ ID NO: 5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 5:
   AATTCTCATT ACCGTTTGAT C
        21
(2) INFORMATION FOR SEQ ID NO: 6:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 6:
   Glu Ile Lys Arg
   1
(2) INFORMATION FOR SEQ ID NO: 7:
   (i) SEQUENCE CHARACTERISTICS:
      (A) LENGTH: 717 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..717
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 239 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Leu Gln Glu Ser Gly Gly His Leu Val Lys Pro Gly Gly Ser Leu Lys
    1               5                  10                  15
   Leu Ser Cys Ala Ala Ser Gly Phe Ala Phe Ser Ser Phe Asp Met Ser
               20                  25                  30
   Trp Ile Arg Gln Thr Pro Glu Lys Arg Leu Glu Trp Val Ala Ser Ile
           35                  40                  45
   Thr Asn Val Gly Thr Tyr Thr Tyr Tyr Pro Gly Ser Val Lys Gly Arg
       50                  55                  60
   Phe Ser Ile Ser Arg Asp Asn Ala Arg Asn Thr Leu Asn Leu Gln Met
   65                  70                  75                  80
   Ser Ser Leu Arg Ser Glu Asp Thr Ala Leu Tyr Phe Cys Ala Arg Gln
                   85                  90                  95
   Gly Thr Ala Ala Gln Pro Tyr Trp Tyr Phe Asp Val Trp Gly Gln Gly
               100                 105                 110
   Thr Thr Val Thr Val Ser Ser Gly Gly Gly Gly Ser Gly Gly Gly Gly
           115                 120                 125
   Ser Gly Gly Gly Gly Ser Asp Ile Glu Leu Thr Gln Ser Pro Lys Ser
       130                 135                 140
   Met Ser Met Ser Val Gly Glu Arg Val Thr Leu Ser Cys Lys Ala Ser
   145                 150                 155                 160
   Glu Thr Val Asp Ser Phe Val Ser Trp Tyr Gln Gln Lys Pro Glu Gln
                   165                 170                 175
   Ser Pro Lys Leu Leu Ile Phe Gly Ala Ser Asn Arg Phe Ser Gly Val
               180                 185                 190
   Pro Asp Arg Phe Thr Gly Ser Gly Ser Ala Thr Asp Phe Thr Leu Thr
           195                 200                 205
   Ile Ser Ser Val Gln Ala Glu Asp Phe Ala Asp Tyr His Cys Gly Gln
       210                 215                 220
   Thr Tyr Asn His Pro Tyr Thr Phe Gly Gly Gly Thr Lys Leu Glu
   225                 230                 235
(2) INFORMATION FOR SEQ ID NO: 9:
   (i) SEQUENCE CHARACTERISTICS:
      (A) LENGTH: 107 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 9:
   AATTCCATGG GCTTCCGATC TCTACTCGCC CTGAGCGGCC TCGTCTGCAC        50
   AGGGTTGGCA CAGGTGCAGC TGCAGTAAGT GACTAAGCTC GAGATCAAAC       100
   GGTGATA                                                      107
(2) INFORMATION FOR SEQ ID NO: 10:
   (i) SEQUENCE CHARACTERISTICS:
      (A) LENGTH: 107 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 10:
   AGCTTATCAC CGTTTGATCT CGAGCTTAGT CACTTACTGC AGCTGCACCT        50
   GTGCCAACCC TGTGCAGACG AGGCCGCTCA GGGCGAGTAG AGATCGGAAG       100
   CCCATGG                                                      107
(2) INFORMATION FOR SEQ ID NO: 11:
   (i) SEQUENCE CHARACTERISTICS:
      (A) LENGTH: 23 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 11:
   Met Gly Phe Arg Ser Leu Leu Ala Leu Ser Gly Leu Val Cys Thr
    1               5                  10                  15
   Gly Leu Ala Gln Val Gln Leu Gln
                   20
(2) INFORMATION FOR SEQ ID NO: 12:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 12:
   Val Thr Lys Leu Glu Ile Lys Arg
   1               5
(2) INFORMATION FOR SEQ ID NO: 13:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 13:
   GCTTCCTCCC TTTTAGACGC AACTGAGAGC CTGAGGTTCA TCCCCAGCAT    50
   CATTACACCT GAGC                                           64
(2) INFORMATION FOR SEQ ID NO: 14:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 14:
   CATGGCTGAG GTGTAATGAT GGTGGGGATG AAGCTCAGGC TCTCAGTTGC    50
   GTCTAAAAGG GAGGAAGC                                       68
(2) INFORMATION FOR SEQ ID NO: 15:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 15:
   CATGGCCGAT ATCGCAAGCT TCCG                                24
(2) INFORMATION FOR SEQ ID NO: 16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 16:
   GATCCGGAAG CTTGCGATAT CGGC 24
(2) INFORMATION FOR SEQ ID NO: 17:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 17:
   AATTCGATAT CGAAGCGCGG CGGATCCCAG GTGCAGCTGC A 41
(2) INFORMATION FOR SEQ ID NO: 18:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 18:
   GCTGCACCTG GGATCCGCCG CGCTTCGATA TCG 33
(2) INFORMATION FOR SEQ ID NO: 19:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 19:
   Ile Ser Lys Arg Gly Gly Ser Gln Val Gln Leu Gln
   1               5                   10
(2) INFORMATION FOR SEQ ID NO: 20:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 20:
   AATTCGATAT CGATCGAAGG TCGAGGCGGA TCCCAGGTGC AGCTGCAG        48
(2) INFORMATION FOR SEQ ID NO: 21:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 21:
   GCTGCACCTG GGATCCGCCT CGACCTTCGA TCGATATCG                   39
(2) INFORMATION FOR SEQ ID NO: 22:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 22:
   Ile Ser Ile Glu Gly Arg Gly Gly Ser Gln Val Gln Leu Gln
    1              5                   10
(2) INFORMATION FOR SEQ ID NO: 23:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 23:
   GGCCGCTGTG CAG                                                    13
(2) INFORMATION FOR SEQ ID NO: 24:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 24:
   AATTCTGCAC AGC                                                    13
(2) INFORMATION FOR SEQ ID NO: 25:
   (i) SEQUENCE CHARACTERISTICS:
      (A) LENGTH: 699 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..699
   (xi) SEQUENCE DESCRIPTION: SEQ ID NO: 25:
(2) INFORMATION FOR SEQ ID NO: 26:
   (i) SEQUENCE CHARACTERISTICS:
      (A) LENGTH: 233 amino acids
      (B) TYPE: amino acid
      (D) TOPOLOGY: linear
   (ii) MOLECULE TYPE: protein
   (xi) SEQUENCE DESCRIPTION: SEQ ID NO: 26:
   Leu Gln Glu Ser Gly Pro Gly Leu Val Ala Pro Ser Gln Ser Leu Ser
    1               5                  10                  15
   Ile Thr Cys Thr Val Ser Gly Phe Ser Leu Thr Gly Tyr Gly Val Asn
               20                  25                  30
   Trp Val Arg Gln Pro Pro Gly Lys Gly Leu Glu Trp Leu Gly Met Ile
           35                  40                  45
   Trp Gly Asp Gly Asn Thr Asp Tyr Asn Ser Ala Leu Lys Ser Arg Leu
       50                  55                  60
   Ser Ile Ser Lys Asp Asn Ser Lys Ser Gln Val Phe Leu Lys Met Asn
   65                  70                  75                  80
   Ser Leu His Thr Asp Asp Thr Ala Arg Tyr Tyr Cys Ala Arg Glu Arg
                   85                  90                  95
   Asp Tyr Arg Leu Asp Tyr Trp Gly Gln Gly Thr Thr Val Thr Val Ser
               100                 105                 110
   Ser Gly Gly Gly Gly Ser Gly Gly Gly Gly Ser Gly Gly Gly Gly Ser
           115                 120                 125
   Asp Ile Glu Leu Thr Gln Ser Pro Ala Ser Leu Ser Ala Ser Val Gly
       130                 135                 140
   Glu Thr Val Thr Ile Thr Cys Arg Ala Ser Gly Asn Ile His Asn Tyr
   145                 150                 155                 160
   Leu Ala Trp Tyr Gln Gln Lys Gln Gly Lys Ser Pro Gln Leu Leu Val
                   165                 170                 175
   Tyr Tyr Thr Thr Thr Leu Ala Asp Gly Val Pro Ser Arg Phe Ser Gly
               180                 185                 190
   Ser Gly Ser Gly Thr Gln Tyr Ser Leu Lys Ile Asn Ser Leu Gln Pro
           195                 200                 205
   Glu Asp Phe Gly Ser Tyr Tyr Cys Gln His Phe Trp Ser Thr Pro Arg
       210                 215                 220
   Thr Phe Gly Gly Gly Thr Lys Leu Glu
   225                 230
(2) INFORMATION FOR SEQ ID NO: 27:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 27:
   AATTCGATAT CGAAGCGCGG CGGATCCCAG GTGCAGCTGC AGTAAGTGAC    50
   TAAGCTCGAG ATCAAACGGT GATAAGCTCG CTTA                     84
(2) INFORMATION FOR SEQ ID NO: 28:
   (i) SEQUENCE CHARACTERISTICS:
      (A) LENGTH: 84 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 28:
   AGCTTAAGCG AGCTTATCAC CGTTTGATCT CGAGCTTAGT CACTTACTGC    50
   AGCTGCACCT GGGATCCGCC GCGCTTCGAT ATCG                     84
(2) INFORMATION FOR SEQ ID NO: 29:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 29:
   Ile Ser Lys Arg Gly Gly Ser Gln Val Gln Leu Gln
   1               5                   10
(2) INFORMATION FOR SEQ ID NO: 30:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 30:
   Val Thr Lys Leu Glu Ile Lys Arg
   1               5
(2) INFORMATION FOR SEQ ID NO: 31:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 31:
   TGTCACGATC TCCTCTTAAG GGATAAGTGC CTTGGTAGTC 40
(2) INFORMATION FOR SEQ ID NO: 32:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 32:
   AGTCGAATTC GATATCACAT TAGCGGATCC GGAGATCGTG ACA 43
(2) INFORMATION FOR SEQ ID NO: 33:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 33:
   Val Thr Ile Ser Ser
   1 5
(2) INFORMATION FOR SEQ ID NO: 34:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 34:
   Gly Ser Ala Asn Val Ile Ser Asn Ser Thr
   1               5                   10
(2) INFORMATION FOR SEQ ID NO: 35:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 35:
   GAACTAACGA ACCGTCCATC                                            20
(2) INFORMATION FOR SEQ ID NO: 36:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 36:
   AATTGCGGCC GC                                                    12
(2) INFORMATION FOR SEQ ID NO: 37:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 37:
   AGGTSMARCT GCAGSAGTCW GG                                         22
(2) INFORMATION FOR SEQ ID NO: 38:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 38:
   TGAGGAGACG GTGACCGTGG TCCCTTGGCC CC 32
(2) INFORMATION FOR SEQ ID NO: 39:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 39:
   GACATTGAGC TCACCCAGTC TCCA 24
(2) INFORMATION FOR SEQ ID NO: 40:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 40:
   GTTTGATCTC GAGCTTGGTC CC 22
(2) INFORMATION FOR SEQ ID NO: 41:
   (i) SEQUENCE CHARACTERISTICS:
      (A) LENGTH: 702 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..702
   (xi) SEQUENCE DESCRIPTION: SEQ ID NO: 41:
(2) INFORMATION FOR SEQ ID NO: 42:
   (i) SEQUENCE CHARACTERISTICS:
      (A) LENGTH: 234 amino acids
      (B) TYPE: amino acid
      (D) TOPOLOGY: linear
   (ii) MOLECULE TYPE: protein
   (xi) SEQUENCE DESCRIPTION: SEQ ID NO: 42:
   Leu Gln Glu Ser Gly Gly Gly Leu Val Gln Pro Gly Gly Ser Arg Lys
    1               5                  10                  15
   Leu Ser Cys Ala Ala Ser Gly Phe Thr Phe Ser Asn Phe Gly Met His
               20                  25                  30
   Trp Val Arg Gln Ala Pro Glu Lys Gly Leu Glu Trp Val Ala Tyr Ile
           35                  40                  45
   Ser Ser Gly Gly Thr Thr Ile Tyr Tyr Ser Asp Thr Met Lys Gly Arg
       50                  55                  60
   Phe Thr Ile Ser Arg Asp Asn Pro Lys Asn Thr Leu Phe Leu Gln Met
   65                  70                  75                  80
   Thr Ser Leu Arg Ser Glu Asp Thr Ala Met Tyr Phe Cys Ala Arg Ser
                   85                  90                  95
   Trp Ala Tyr Ala Met Asp Tyr Trp Gly Gln Gly Thr Thr Val Thr Val
               100                 105                 110
   Ser Ser Gly Gly Gly Gly Ser Gly Gly Gly Gly Ser Gly Gly Gly Gly
           115                 120                 125
   Ser Asp Ile Glu Leu Thr Gln Ser Pro Ser Tyr Leu Ala Ala Ser Pro
       130                 135                 140
   Gly Glu Ile Ile Thr Ile Asn Cys Arg Ala Ser Lys Ser Ile Ser Lys
   145                 150                 155                 160
   Tyr Leu Ala Trp Tyr Gln Glu Lys Pro Gly Lys Thr Asn Lys Leu Leu
                   165                 170                 175
   Ile Tyr Ser Gly Ser Ile Leu Gln Ser Gly Ile Pro Ser Arg Phe Ser
               180                 185                 190
   Gly Ser Gly Ser Gly Thr Asp Phe Thr Leu Thr Ile Ser Ser Leu Glu
           195                 200                 205
   Pro Glu Asp Phe Ala Met Tyr Tyr Cys Gln Gln His Asn Glu Tyr Pro
       210                 215                 220
   Trp Thr Phe Gly Gly Gly Thr Lys Leu Glu
   225                 230
(2) INFORMATION FOR SEQ ID NO: 43:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 43:
   CATGCAGTCT TCGGGC                                                    16
(2) INFORMATION FOR SEQ ID NO: 44:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 44:
   TTAAGCCCGA AGACTG                                                    16
(2) INFORMATION FOR SEQ ID NO: 45:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 45:
   Asn Val Ile Ser Lys Arg
   1               5

## Description

The present invention relates to the production of a Single Chain antibody fragment (ScFv fragment) by a transformed mould. In this specification an ScFv fragment stands for a variable fragment of a heavy chain connected by a linker peptide to a variable fragment of a light chain.

### Background of the invention

It has been described that ScFv fragments can be produced in various transformed microorganisms, but with various degrees of success. For example, from WO 93/02198 (TECH. RES. INST. FINLAND; Teeri c.s.) published 04.02.93 it is known that ScFv fragments can be produced and secreted in several host organisms (although it is only exemplified in *E. coli* and *S. cerevisiae),* provided that a special linker is used between the heavy chain and the light chain fragments. That linker comprises a flexible hinge region of a naturally secreted multidomain protein or an analogue thereof not being homologous to either of the heavy or light chain fragments. This WO 93/02198 is incorporated herein by reference. A serious limitation of the method disclosed in WO 93/02198 is the low production level shown, which is far below the production level required for the application of ScFv fragments in consumer products at a reasonable price. Examples of such consumer products include detergent products, food products, and products for the personal care of people like toilet soap and under arm hygienic products. Thus there is a need for a more universal high-yielding production system for ScFv fragments. The production of an ScFv fragment in *E. coli* **bacteria** gives relatively low yields and there is a need for solubilization and subsequent renaturation of the proteins formed inside the bacteria, which makes this method not attractive for production of antibody fragments that need be used in relatively large amounts (see page 3, lines 5-23 of WO 93/02198). When attempting to produce various ScFv fragments in yeasts using expression systems, that have produced various heterologous enzymes in amounts sufficient for economical application in consumer goods, the present inventors found that the ScFv fragments were not secreted or only in very minute quantities. This appears to be in agreement with Example 2 on pages 29-31 of WO 93/02198 which relates to the production of an ScFv fragment in yeast without indicating the amount produced. Although in WO 93/02198 many alternative linkers are mentioned, it is stated on page 6 of WO 93/02198 that "... there are no published reports of the analysis or design of secretable linker peptides." and "... there are no published examples to date of novel fusion proteins with added heterologous linker sequences which are secreted to the culture medium of the host."

In another recent publication, namely in WO 92/01797 (OY ALKO AB), published 06.02.92, the production of immunoglobulins in the mould *Trichoderma* is described. In Example 20 on pages 83-85 and Figure 27 the construction and expression of a functional gene encoding a single chain antibody containing variable regions of both a light and heavy chain linked to each other by a flexible hinge region of CBHI is described (CBHI is cellobiohydrolase I present in large amounts in the culture medium of *Trichoderma reesei;* see page 3 of WO 92/01797). The gene was under control of a *T. reesei cbhi* terminator and either a *T. reesei cbhi* promoter (plasmid pEN401) or an *Aspergillus gpd* promoter (plasmid pEN402). The plasmids were transformed to *Trichoderma reesei* strain RUT-C-30 (ATCC 56765) and the transformants were grown in two different media. Expression of immunoreactive single chain antibodies was tested from culture supernatants but no results were mentioned. **Thus it was not demonstrated that any amount of single chain antibodies was actually formed.** This conclusion is in agreement with a later related publication of Nyyssönen *et al.* ex VTT Biotechnical Laboratory, Finland (1993) in which partially the same experiments are described with plasmids pEN304, pAJ202 and pEN209 encoding the 23.3 kD light chain, the 23.9 kD heavy Fd chain and the 73.2 kD CBHI-heavy Fd chain, respectively, which plasmids are also exemplified in WO 92/01797. In this publication only the production of a separate light chain or a separate heavy chain, as such or as a precursor, by a *Trichoderma reesei* strain is described, but the production of an ScFv fragment containing a light chain connected via a linker peptide to a heavy chain is not described.

**Therefore, there is still a need for an alternative production and secretion system for ScFv fragments in a mould that gives at least a reasonable yield of the desired ScFv fragment.** The present invention provides such production using a transformed mould of the genus *Aspergillus.*

According to M. Ward *et al.* (1990), see also GENENCOR's WO 90/15860 published 27.12.90, the production in *Aspergillus* of a desired protein and subsequent secretion can be improved when a fusion protein comprising the desired protein and a mould protein is produced. This was exemplified with the production of prochymosin fused with its amino terminus to the carboxyl terminus of *A. awamori* glucoamylase. However, that publication does not give any suggestion that such an approach would also be suitable for the production of ScFv fragments, which are known as compounds presenting great difficulties when one attempts to obtain their production and secretion by a microbial host (see the above mentioned WO 93/02198).

In UNILEVER's not prior-published WO 93/12237, now published 24.06.93 and claiming a priority date of 09.12.91, a process for the production and secretion of a desired protein by a transformed mould is described, in which the expression and/or secretion regulating regions are derived from the endoxylanase II gene (*exlA* gene) of *Aspergillus niger* var. *awamori* present on plasmid pAW14B (see Figure 3 of WO 93/12237), which is present in a transformed E. *coli* strain JM109 deposited under the Budapest Treaty at the Centraalbureau voor Schimmelcultures in Baarn, The Netherlands, as N° CBS 237.90 on 31 May 1990. In a preferred embodiment the desired protein can be part of a fusion protein comprising the desired protein preceded at its NH₂-terminus by at least part of the endoxylanase II protein. No mention is made of the production of ScFv fragments.

### Summary of the invention

The present invention provides a process for producing fusion proteins comprising ScFv fragments by a transformed mould, in which (a) the mould belongs to the genus *Aspergillus*, and (b) the *Aspergillus* contains a DNA sequence encoding the ScFv fragment under control of at least one expression and/or secretion regulating region derived from a mould selected from the group consisting of promoter sequences, terminator sequences and signal sequence-encoding DNA sequences, and functional derivatives or analogues thereof, optionally followed by a proteolytic cleavage step for separating the ScFv fragment part from the fusion protein. In one embodiment the "at least one expression and/or secretion regulating region derived from a mould" comprises the combination of both a promoter sequence and a signal sequence-encoding DNA sequence derived from a glucoamylase gene ex *Aspergillus* plus a terminator sequence of a *trpC* gene ex *Aspergillus* or at least one functional derivative or analogue thereof. In another embodiment the "at least one expression and/or secretion regulating region derived from a mould" is selected from a promoter, a signal sequence-encoding DNA sequence and a terminator sequence derived from an endoxylanase gene ex *Aspergillus,* especially from the endoxylanase II gene (*exlA* gene) of *Aspergillus niger* var. *awamori* present on the above mentioned plasmid pAW14B or at least one functional derivative or analogue thereof.

In a preferred embodiment of the present invention the DNA sequence encoding the ScFv fragment forms part of a chimeric gene encoding a fusion protein, whereby said DNA sequence encoding the ScFv fragment is preceded at its 5' end by at least part of a structural gene encoding the mature part of a secreted mould protein, especially a mature *Aspergillus* protein, e.g. the mature glucoamylase protein or the mature endoxylanase protein. If the ScFv fragment in the fusion protein is connected or bound to said secreted mould protein or part thereof by a proteolytic cleavage site, e.g. a KEX2-like site, it is possible to remove the mould protein or part thereof from the ScFv fragment, so that the resulting antibody fragment is as small as possible, which can have significant advantages in applications. In this case the process according to the invention includes a proteolytic cleavage step for separating the ScFv fragment part from the fusion protein following the production of the fusion protein containing the ScFv fragment. It was found that production levels of at least 40 mg ScFv fragment per litre, or even at least 60 mg/l, and a highest yield of slightly more than 90 mg/l could be obtained (see Table 2 below), but it is envisaged that after further optimization at least 150 mg/l can be achieved by cultivation in shaked flasks. Further, production levels of more than 150 mg ScFv fragment per litre were already obtained with cultivation in a fermenter; it is therefore envisaged that after further optimization at least 250 mg/l, or even at least 500 mg/l, and probably more than at least 1 g/l will be obtainable .

The invention also provides new products comprising an ScFv fragment or fusion product thereof obtainable by a process according to the invention. Such new product can be one in which the ScFv fragment is a modified ScFv fragment comprising complementary determining regions (CDRs) grafted on the framework regions of the variable fragments of an other ScFv fragment that is well expressed and secreted by a lower eukaryote, especially a mould of the genus *Aspergillus.* The invention also provides a composition, in particular consumer products of which examples are given above, containing a product produced by a process according to the invention or a new product as described above. According to a special embodiment of the invention the ScFv fragment recognizes a compound present in the human eco-system, which compound can be a microorganism, an enzyme or another protein. One preference is for compounds present in the oral cavity, and more preferably for compounds involved in the formation of plaque, caries, gingivitis, periodontal diseases, or bad breath. Another preference is for compounds present on the human skin, more preferably compounds involved in the formation of malodour, inflammation or hair loss. Another special embodiment of the invention relates to a composition, which can be used for diagnostic purposes and in which the compound is a hormone, especially human chorionic gonadotropin (HCG).
According to another embodiment of the invention the ScFv fragment recognizes a compound present in the eco-system of domestic and agricultural animals which compound can be an animal feed component, an enzyme or another protein, or a disease causing agent.
According to still another embodiment of the invention a composition is provided in which the ScFv fragment recognizes a compound that has a positive or negative relationship with a disease or disorder and can for example be used for detection and/or targeting purposes.
The invention also relates to a composition according to the invention which can be used in the chemical, petrol or pharmaceutical industry as a catalyst or for detection purposes.
Although the invention was developed on the basis of the production of ScFv fragments in a mould of the genus *Aspergillus,* as will be illustrated in the Examples below, it is envisaged that the invention will also be applicable to other moulds, especially selected from the genera *Mucor, Neurospora,* and *Penicillium.*

### Brief description of the figures

- Figure 1: Schematic drawing of pAN52-10.
- Figure 2: Schematic drawing of pUR4155 and pUR4157.
- Figure 3: Schematic drawing of pAN56-7.
- Figure 4: Schematic drawing of pUR4159 and pUR4161.
- Figure 5: Western blot. After gelelectrophoresis on a 12.5% SDS-PAGE gel proteins reacting with Fv-lysozyme antiserum are visualized. Lane 1: *E. coli* extract containing ScFv-lysozyme; Lane 2: Fv-lysozyme; Lanes 3 to 8 contain medium samples of AWC(M)41 transformants and the A. *niger* var. *awamori* mutant #40 strain; Lane 3 and 4: transformant AWC(M)4161 (prepro-"glaA2"-KEX-ScFv-HCG); Lane 5: AWC4159 (prepro-"glaA2"-KEX-ScFv-LYS); Lane 6: mutant #40; Lane 7: AWC4157 (18aa glaA-ScFv-HCG); Lane 8: AWC4155 (18aa glaA-ScFv-LYS).
- Figure 6: Map of plasmid pAW14B obtained by insertion of the 5.3 kb *Sai*l fragment comprising the *exlA* gene of *Aspergillus niger* var. *awamori* in the *Sal*I site of pUC19.
- Figure 7: Coomassie Brilliant Blue-stained polyacrylamide gel showing proteins present in the culture medium of an *Aspergillus niger* var. *awamori* transformed with pUR4462; also indicated are the bands representing
(i) the released ScFv-LYS fragment, and
(ii) the glaA-KEX2-ScFv-LYS fusion protein and/or the truncated glaA protein.

### Detailed description of the invention

It has now been found that the development described above by M. Ward *et al.* (1990) and in WO 90/15860 (in which the gene encoding the desired protein forms part of a chimeric gene further comprising a gene encoding the glucoamylase protein) as well as the above described preferred embodiment of the invention described in UNILEVER's above mentioned not prior-published WO 93/12237 (in which the gene encoding the desired protein forms part of a chimeric gene further comprising a gene encoding at least part of the endoxylanase protein) can be applied advantageously for the production of ScFv fragments, so that the desired protein is the ScFv fragment. This is particularly so, when in the resulting fusion protein a proteolytic cleavage site is present between the secreted mould protein part or fragment thereof and the ScFv part. A preferred cleavage site is a KEX2-like site as described by Fuller *et al.* (1988), Contreras *et al.* (1991) and Calmels *et al.* (1991), but other cleavage sites can also be used provided that they are not present in the ScFv fragment. Other cleavage sites can be selected on the basis of the method described by Matthews & Wells (1993). In the Examples given below the pro part of the prepro-glucoamylase protein comprises a KEX2-type recognition site, see Example 2.4 (i).

ScFv fragments that recognize microorganisms present in the oral cavity or on the skin of human beings are important in the framework of this invention, because they have potential to inhibit the growth or metabolism of these microorganisms. Certain microorganisms present in the oral cavity are thought to be involved in the formation of plaque, caries, gingivitis or periodontal diseases, etc., whereas microorganisms on the human skin are involved in, amongst others, the generation of malodour. The ScFv fragments prepared according to the invention may exert their action either as such, or bound to other compounds that have an inhibitory effect on said microorganisms.

It is also envisaged that according to the present invention other modified ScFv fragments can be made by grafting a complementary determining region (CDR) on the framework regions of the variable fragments of an ScFv fragment that is well expressed and secreted in *Aspergillus;* compare grafting of CDR's on human immunoglobulins as described by e.g. Jones *et al.,* (1986). These CDR's can be obtained from common antibodies. Both the binding properties of a CDR and the remainder of the ScFv fragment can be optimized by random or directed mutagenesis. Thus in a process according to the invention CDR's originating from one antibody can be grafted on the framework regions of the variable fragments of another ScFv fragment.

Some ScFv fragments or fusion products thereof produced by a process according to the invention may be old, but many of the ScFv fragments or fusion products thereof will be new products. Thus the invention also provides new ScFv fragments or fusion products thereof obtainable by a process according to the invention. The products resulting from such process can be used in compositions for various applications. Therefore, the invention also relates to compositions containing a product produced by a process according to the invention. This holds for both old products and new products.

Instead of the combination of an *exlA* promoter, an exlA signal sequence-encoding DNA sequence, and an *exlA* terminator exemplified in Examples 3 and 5, also other combinations can be used e.g. an *exlA* promoter, an glaA signal sequence-encoding DNA sequence, and an *exlA* terminator as exemplified in Example 7, but in general a selection can he made from any mould-derived promoter, mould-derived signal sequence-encoding DNA sequence, and mould-derived terminator sequence as expression and/or secretion regulating regions. A specific embodiment is a combination of both a promoter sequence and a signal sequence-encoding DNA sequence derived from a glucoamylase gene ex *Aspergillus* plus a terminator sequence of a *trp*C gene ex *Aspergillus.*
The secreted mould protein forming part of a fusion protein according to the invention can in general he derived from any secreted mould protein in addition to the exemplified endoxylanase II protein ex *Aspergillus niger* var. *awamori* (see Examples 3 and 5) and the exemplified glucoamylase ex *Aspergillus* (see Example 7).
Table 2 in Example 2.6.1b shows that the highest expression and secretion yield was obtained when the mould protein was composed of its prepro part followed by an appreciable part of its mature protein, which was connected to the ScFv fragment by again the pro part of the mould protein containing a KEX2-like cleavage site. A small linker peptide may be situated between the ScFv fragment and the KEX2-like cleavage site (see plasmids pUR4159 and pUR4163 and derivatives) or between the latter and the part of the mature mould protein. Thus in its broadest sense the invention provides a process for producing fusion proteins comprising ScFv fragments by a transformed mould, in which the mould belongs to the genus *Aspergillus,* and the *Aspergillus* contains a DNA sequence encoding the ScFv fragment under control of at least one expression and/or secretion regulating region derived from a mould selected from the group consisting of promoter sequences, terminator sequences and signal sequence-encoding DNA sequences, or functional derivatives or analogues thereof.

The invention will be illustrated by the following Examples.

### Example 1 Isolation of the antibody gene fragments encoding the V_{H} and V_{L} regions and the construction of ScFv genes.

The isolation of RNA from the hybridoma cell lines, the preparation of cDNA and amplification of gene fragments encoding the variable regions of the heavy (V_{H}) and light (V_{L}) chains of the antibodies by PCR, was performed according to standard procedures known from the literature (see e.g. Orlandi *et al,* 1989). The general procedures described in the Examples were performed according to Sambrook *et al.,* unless otherwise indicated.
After cloning the V_{H} and V_{L} gene fragments and determining the nucleotide sequence, they can be used to construct expression plasmids encoding e.g. Fv or ScFv antibody fragments. In the ScFv antibody fragments, the V_{H} and the V_{L} chains are connected via a peptide linker. This is achieved by constructing a (chimeric) gene in which the gene fragments encoding the V_{H} and V_{L} chains are connected with a nucleotide sequence encoding the linker peptide. The order of the variable chains can be V_{H}-linker-V_{L} or V_{L}-linker-V_{H}. In the following experiments the peptide linker with the sequence (GGGGS)₃ is used (SEQ. ID. NO: 1).

### 1.1 Construction of ScFv anti-lysozyme

Plasmid pScFv-LYS-myc was obtained from G. Winter and was described by S. Ward *et al.,* (1989). This pUC19-derived plasmid contains a gene fragment encoding the V_{H} and V_{L} fragments of the anti-Hen egg white lysozyme antibody D1.3. The V_{H} fragment is preceded by the PelB secretion signal sequence, the V_{H} and V_{L} fragments are connected via the (GGGGS)₃ peptide linker (SEQ. ID. NO: 1) and the V_{L} fragment is extended with an 11 amino acids myc-tag. The nucleotide sequence (SEQ. ID. NO: 2) and the deduced amino acid sequence (SEQ. ID. NO: 3) of the *Hin*dIII-*Eco*RI fragment encoding the ScFv fragment of the monoclonal anti-lysozyme antibody D1.3, preceded by the PelB signal sequence and followed by the myc-tail are given below.

In order to remove the myc-tag of pUC19-derived pScFv-LYS-myc the *Xho*I-*Eco*RI fragment was replaced by a new synthetic fragment having the following sequence :

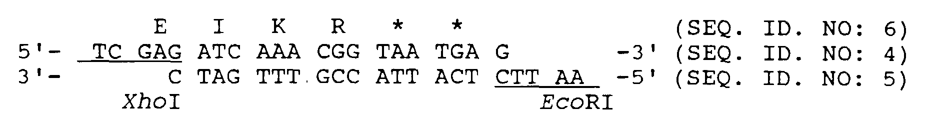

introducing a TAA translation termination codon after the V_{L}-gene fragment. The obtained plasmid was named pUR4121. Subsequently, the about 820 bp *Hin*dIII-*Eco*RI fragment encoding the ScFv-LYS was isolated and cloned into a pEMBL9-derived plasmid (Dente *et al.,* 1983), which was digested with the same enzymes, resulting in plasmid pUR4129.

### 1.2 Construction of a gene encoding ScFv anti-human chorionic gonadotropin

Human chorionic gonadotropin (HCG) is a pregnancy hormone. A pregnancy test kit based on the detection of HCG in urine by using monoclonal antibodies was developed by Unilever and is marketed by UNIPATH under the trade name Clearblue®. Gene fragments, encoding the variable regions of the heavy and light chain fragments from the monoclonal antibody directed against the human chorionic gonadotropin were obtained from a hybridoma cell line in a way as described above. Subsequently, these HCG V_{H} and V_{L} gene fragments were cloned into plasmid pUR4129 by replacing the corresponding *Pst*I-*Bst*EII and *Sac*I*-Xho*I anti-lysozym gene fragments, resulting in plasmid pUR4138. The nucleotide sequence (SEQ. ID. NO: 7) and the deduced amino acid sequence (SEQ. ID. NO: 8) of the *Pst*I*-Xho*I gene fragment encoding the ScFv fragment of the anti-human chorionic gonadotropin (anti-HCG) antibody is given below.

### Example 2 Construction of ScFv expression cassettes, using the glaA promoter system and introduction into Aspergillus.

### 2.1 Construction of ScFv expression cassettes using the 18 amino acid signal sequence of glucoamylase (pUR4155 and pUR4157)

The multiple cloning site of plasmid pEMBL9 (ranging from the *Eco*RI to the *Hin*dIII site) was replaced by a synthetic DNA fragment having the following nucleotide sequence.

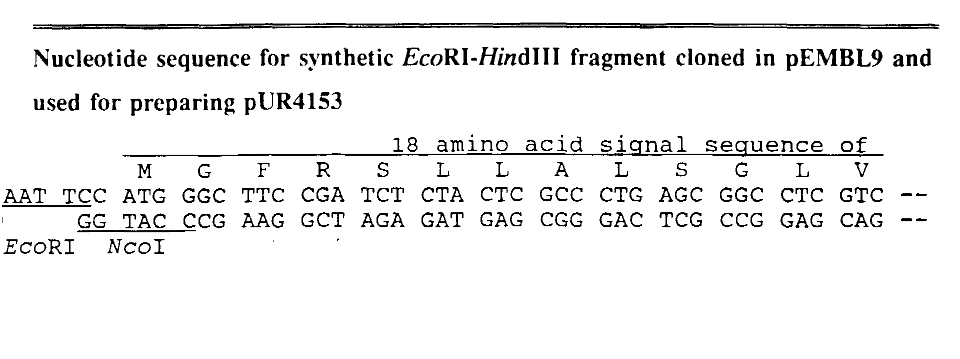

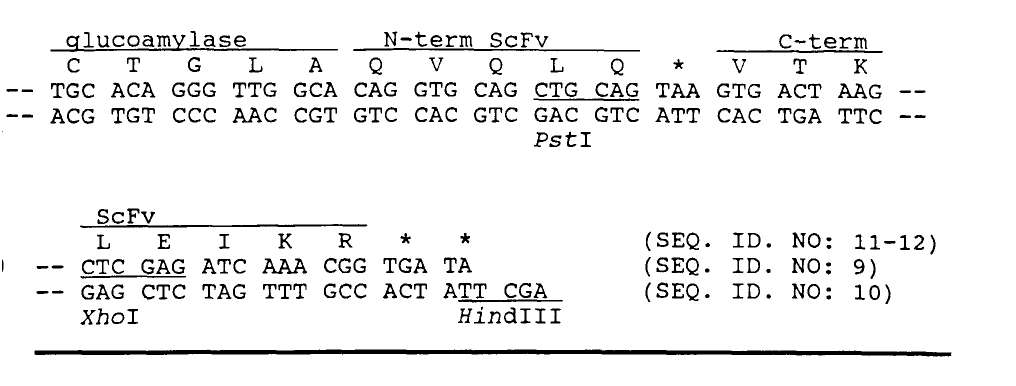

The 5'-part of the nucleotide sequence codes for the glaA signal sequence (amino acid 1 to 18), followed by the first 5 amino acids of the variable part of the antibody heavy chain. The 3'-part encodes the last 5 amino acid residues of the variable part of the antibody light chain. The resulting plasmid was named pUR4153.
Plasmids pUR4154 and pUR4156 were obtained in the following way: After digestion of plasmid pUR4129 (Example 1.1) with *Pst*I and *Xho*I*,* an about 0.7 kb DNA fragment was isolated from agarose gel. This fragment codes for a truncated ScFv-LYS fragment missing DNA sequences encoding the 5 N-terminal and 5 C-terminal amino acids. In the same way an about 0.7 kb *Pst*I*-Xho*I fragment was isolated from plasmid pUR4138 (Example 1.2), which encodes for a similarly truncated ScFv-HCG fragment.
In order to fuse the ScFv encoding fragments with the glaA secretion signal-encoding sequence, the obtained fragments were cloned into pUR4153. To this end plasmid pUR4153 was digested with *Pst*I and *Xho*I*,* after which the about 4.1 kb vector fragment was isolated from an agarose gel. Ligation with the about 0.7 kb *Pst*I*-Xho*I fragments resulted in plasmids pUR4154 (ScFv-LYS) and pUR4156 (ScFv-HCG), respectively.

### 2.2 Construction of pAN52-10

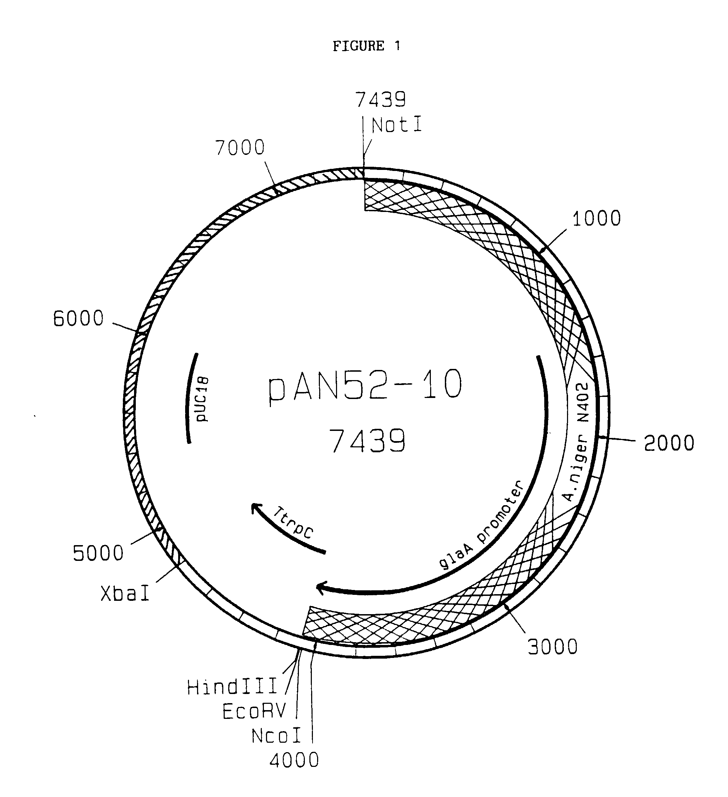

pAN52-10 (**Figure 1**) was used as starting vector for the construction of the *Aspergillus* expression cassettes. This plasmid was constructed as follows: In pAN52-6*Not*I (Van den Hondel *et al.,* 1991) the *Nco*I site located in the *glaA* promoter of *A*. *niger* N402 (about 2.7 kb upstream of the ATG) was removed by cleaving with *Nco*I and filling in with Klenow polymerase, resulting in pAN52-*6Not*I delta *Nco*I*.* After digestion of pAN52-6*Not*I delta *Nco*I with *Not*I and partial digestion with *Xmn*I an about 4.0 kb *Not*I*-Xmn*I *glaA* promoter fragment was isolated. Three-way ligation of this pAN52-6*Not*I delta *Nco*I fragment (1) with an about 3.4 kb *Not*I*-Nco*I fragment (2) of pAN52-1*Not*I (Van den Hondel, C.A.M.J.J. *et al.;* 1991), comprising the *A. nidulans trpC* terminator (Punt, J.P. *et al.;* 1991) and pUC18-sequences, and with a synthetic *Xmn*I*-Nco*I fragment (3) comprising the 3'-end of the *glaA* promoter to the ATG initiation codon, resulted in plasmid pAN52-*7Not*I. The nucleotide sequence (SEQ. ID. NO: 13-14) of this synthetic *Xmn*I*-Nco*I fragment is given below.

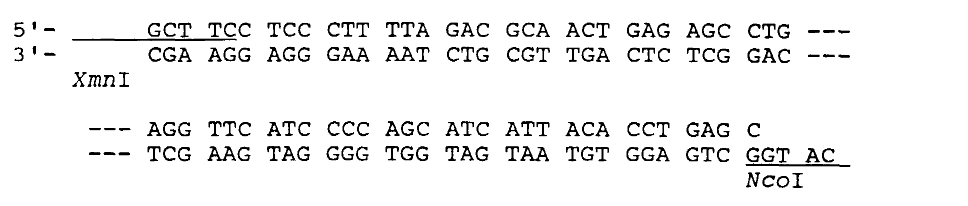

After isolating both the about 4 kb *Not*I*-Nco*I fragment (comprising the *glaA* promoter) and the about 3.4 kb *Not*I*-Bam*HI fragment (comprising the pUC18 vector and the *trpC* terminator) from pAN52-7*Not*I, the fragments were ligated together with the *Nco*I*-Bam*HI linkers containing an *Eco*RV site and an *Hin*dIII site and having the following nucleotide sequences (SEQ. ID. NO: 15-16).

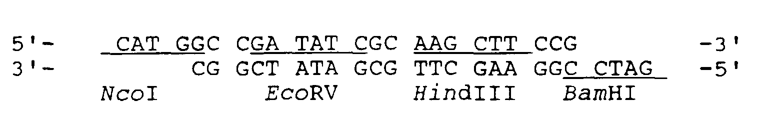

This resulted in plasmid pAN52-9. Ligation of the about 4.0 kb *Not*I-*Hin*dIII *gla*A promoter fragment of pAN52-9 with an about 3.3 kb *Hin*dIII-*Not*I fragment of pAN52-6*Not*I containing both pUC18-sequences and an about 0.7 kb *trpC* terminator fragment of *A. nidulans* resulted in pAN52-10 (**Figure 1**).

### 2.3 Construction of pUR4155 and pUR4157.

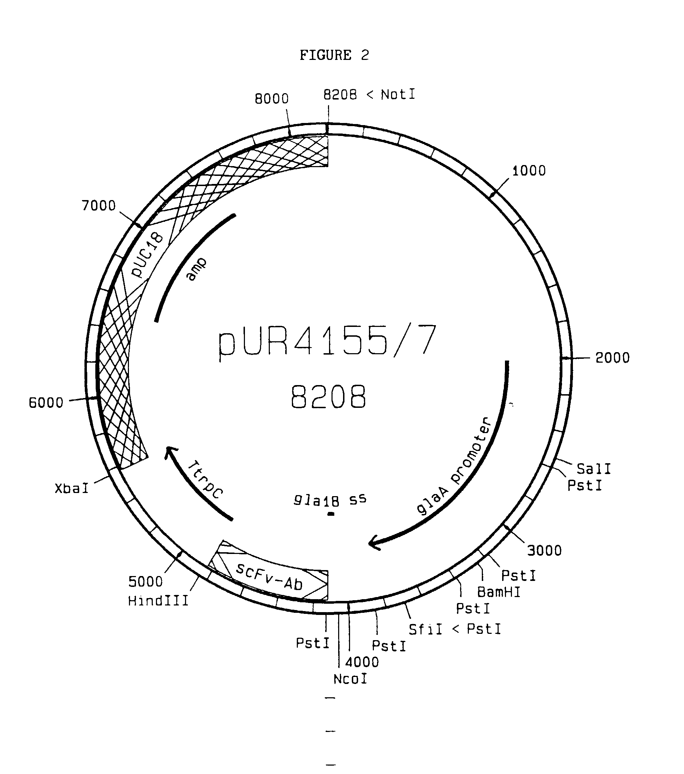

Plasmid pAN52-10 was digested with *Nco*I and *Hin*dIII and the dephosphorylated vector fragment of about 7.5 kb was isolated. The *Nco*I site is located downstream of the *glaA* promoter and coincides with the ATG initiation codon. The plasmids pUR4154 and pUR4156 (see Example 2.1) were digested with *NcoI* and *Hin*dIII and the about 0.8 kb fragments coding for the ss-glaA and the ScFv were isolated. Ligation of the obtained fragments resulted in plasmids pUR4155 and pUR4157, respectively (**Figure 2**). In these plasmids the expression of the ScFv fragments is under the control of the *A. niger glaA* promoter, the 18 amino acid signal sequence of glucoamylase and the *A. nidulans trpC* terminator.

### 2.4 Construction of ScFv expression cassettes using part of glucoamylase as a secretion carrier.

### i) Construction of pUR4159 and pUR4161.

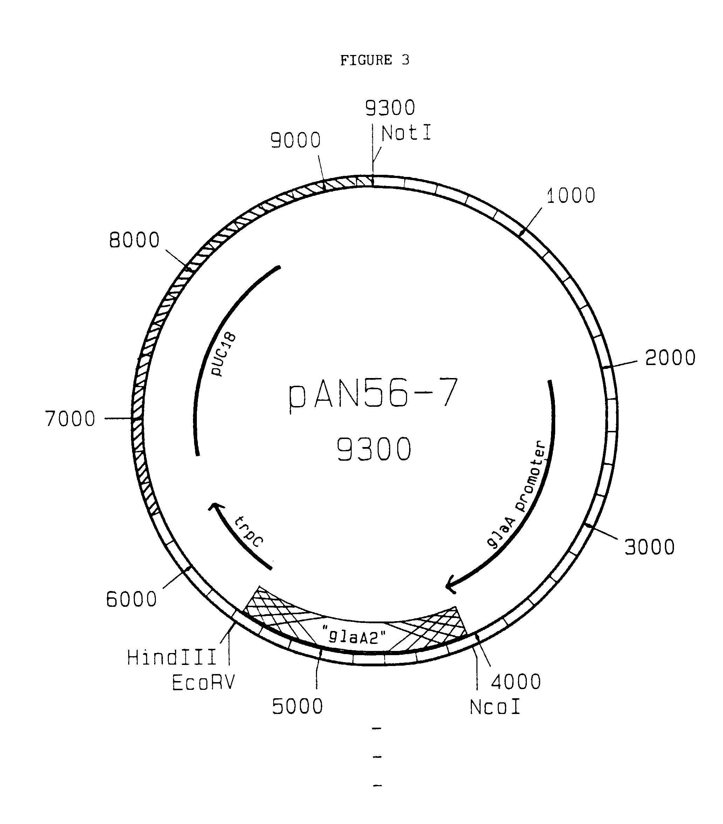

Expression cassettes encoding a fusion protein consisting of the glaA prepropart, the first 514 amino acids of the mature glucoamylase G1 protein ("glaA2" protein), and the ScFv fragments were constructed. In these cassettes the "glaA2" protein and the ScFv fragment were intersected by a sequence which encodes the propeptide of glucoamylase (Asn-Val-Ile-Ser-Lys-Arg; SEQ. ID. NO: 45) and which comprises a KEX2-type recognition site (Lys-Arg). To obtain these vectors, plasmid pAN56-7 (**Figure 3**) was constructed by insertion of a 1.9 kb *Nco*I*-Eco*RV fragment of pAN56-4, comprising part of the A. *niger glaA* gene into the about 7.5 kb *Nco*I-*Eco*RV fragment of pAN52-10. Plasmid pAN56-4 was not prior-published but its description is now available in the publication of M.P. Broekhuijsen, I.E. Mattern, R. Contreras, J.R. Kinghorn & C.A.M.J.J. van den Hondel in Journal of Biotechnology 31, No.2 (1993) 135-145, which is incorporated herein by reference; a copy of the draft paper was attached to the priority documents.
To obtain in-frame fusions of the "glaA2" protein and the ScFv fragments plasmids pUR4154 and pUR4156 were digested with *Eco*RI and *Pst*I*,* after which the vector fragment of about 4.8 kb was isolated from an agarose gel. The vector was ligated with a synthetic *Eco*RI*-Pst*I fragment having the following nucleotide sequence (SEQ. ID. NO: 17-19).

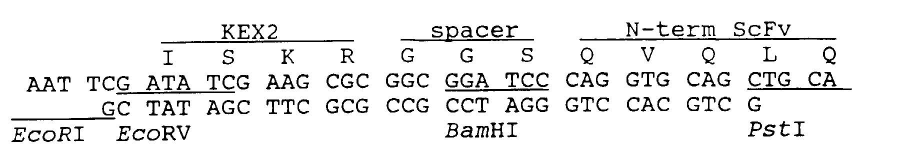

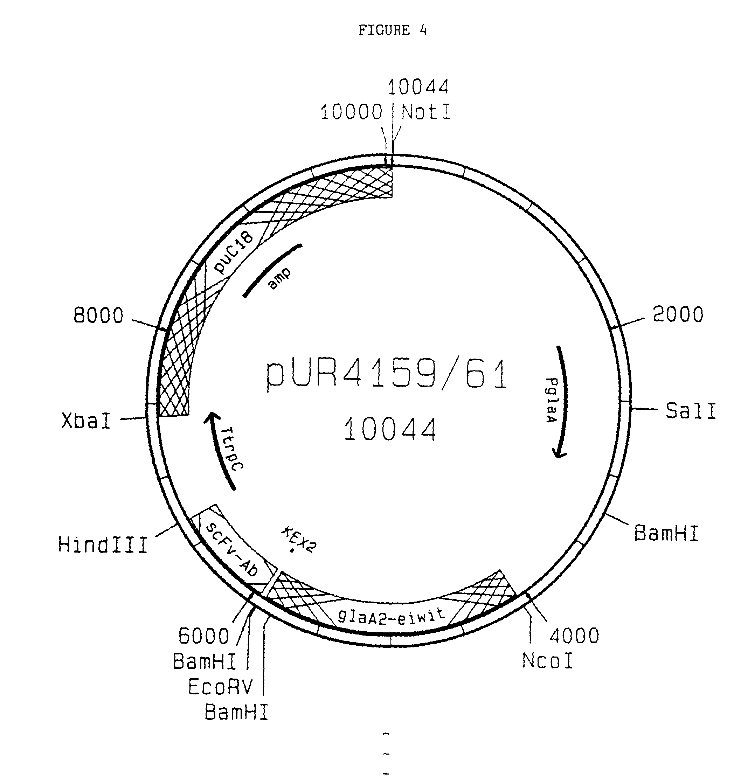

This *Eco*RI*-Pst*I fragment was used to replace the fragment encoding the glaA signal sequence (see Example 2.1) and to allow an in-frame fusion to the *"glaA2"* gene. From the resulting plasmids, pUR4158 and pUR4160, the *Eco*RV-*Hin*dIII fragments (about 0.75 kb) were isolated and ligated into the *Eco*RV-*Hin*dIII fragment of pAN56-7 (about 9.3 kb), resulting in pUR4159 and pUR4161 (**Figure 4**, in which the DNA encoding the 24 amino acid prepro glaA part in the neighbourhood of the *Nco*I site was not indicated). In the resulting protein the "glaA2" part and the ScFv part are connected by a peptide comprising a KEX2 cleavage site.

### ii) Construction of pUR4163.

In a similar way a vector was constructed with an expression cassette encoding a fusion protein consisting of the "glaA2" protein (preceded by its prepro part) fused to ScFv-lysozyme and intersected by a factor Xa recognition site. The *Eco*RI*-Pst*I vector fragment (about 4.8 kb) of pUR4154 was ligated with a synthetic *Eco*RI*-Pst*I fragment having the following nucleotide sequence (SEQ. ID. NO: 20-22).

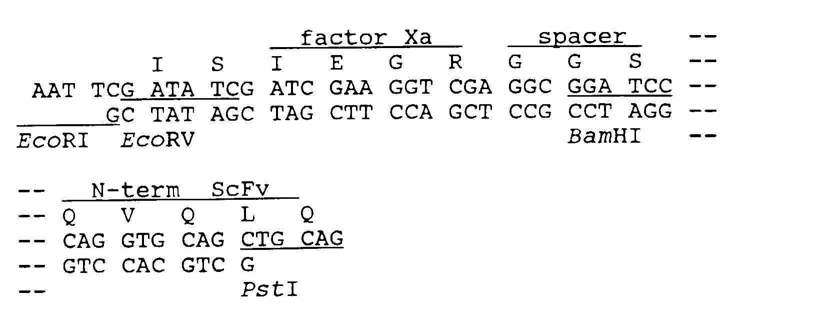

This *Eco*RI-*Pst*I fragment was used to replace the fragment encoding the glaA signal sequence and to allow an in-frame fusion to the "*glaA2*" gene. In the encoded protein the "glaA2" part and the ScFv part are connected by a peptide comprising a factor X cleavage site. From the resulting plasmid pUR4162, the *Eco*RV-*Hind*III fragment (about 0.75 kb) was isolated and ligated into the pAN56-7 vector fragment (about 9.3 kb), resulting in pUR4163.

### 2.5 Aspergillus transformation

The constructed vectors can be provided with conventional selection markers (e.g. *amdS or pyrG,* hygromycin etc.) and the fungus can be transformed with the resulting vectors to produce the desired protein.

**Table 1**

| Expression vectors for the production of ScFv-anti-lysozym and ScFv-anti-human chorionic gonadotropin, resp., controlled by the *A. niger glaA* promoter and *A. nidulans trpC* terminator with *A. nidulans amdS* as selection marker | | | |
|---|---|---|---|
| Plasmids | ScFv-antibody | secretion-carrier | cleavage of ScFv-antibody by |
| pUR4155 | ScFv-LYS | 18 a.a. ss glaA | signalpeptidase |
| pUR4159 | ScFv-LYS | prepro-"glaA2" | KEX2-enzyme |
| pUR4163 | ScFv-LYS | as in pUR4159 | factor Xa |
| pUR4157 | ScFv-HCG | as in pUR4155 | signalpeptidase |
| pUR4161 | ScFv-HCG | as in pUR4159 | KEX2-enzyme |

As an example, the *Aspergillus nidulans amdS* gene (Hynes M.J. **et al.** 1983) located on a 5.0 kb *Not*I fragment was introduced in the unique *Not*I sites of the ScFv expression vectors pUR4155, pUR4157, pUR4159, pUR4161 and pUR4163 yielding pUR4155NOT, pUR4157NOT, pUR4159NOT, pUR4161NOT and pUR4163NOT, respectively (**Table 1)**. The *amdS Not*I fragment was obtained by flanking the *Eco*RI fragment of pGW325 (Wernars K.; Ph.D. thesis 1986) with the following synthetic oligonucleotides. The constructed pUR41..NOT vectors (pUR4155NOT, pUR4157NOT, pUR4159NOT, pUR4161NOT and pUR4163NOT) were subsequently transferred to *Aspergillus niger* var. *awamori* ATCC 11358 ( = CBS 115.52) and a mutant strain *Aspergillus niger* var. *awamori* # 40 (WO 91/19782) which has been obtained by mutagenesis of *A. niger* var. *awamori.* Transformation with pUR41NOT plasmids was carried out as described in WO 91/19782 or by means of co-transformation with plasmid pAN7-1 according to Punt P.J. and Van den Hondel C.A.M.J.J. (1992). pAN7-1 comprises the hygromycin resistance gene of *E. coli* flanked by *Aspergillus* expression signals. The yield of *A. niger* var. *awamori* (mutant #40) protoplasts was 1-5 10⁷/g mycelium and the viability was 3-8%. Per transformation 3-8 x 10⁵ viable protoplasts were incubated with 10 µg plasmid DNA purified by the Qiagen method. *A. niger* var. *awamori* mutant #40 AmdS⁺ transformants were selected and purified on plates with minimal medium and acetamide or acrylamide as sole nitrogen source. Direct selection resulted in up to 0.02 mutant #40 transformants per µg DNA. No *A. niger* var. *awamori* transformants were obtained. Co-transformation of the mutant #40 strain was performed with a mixture of one of the pUR41..NOT plasmids and pAN7-1 DNA in a weight ratio of 7:3. pAN7-1 co-transformants were selected primarily on minimal medium plates containing 100-150 µg/ml hygromycin, followed by selection on plates with acetamide. The frequency of Hm^{R} colonies was about 2 transformants per µg, however only 5% of the Hm^{R} colonies grew well on plates with acetamide.
*A. niger* var. *awamori* mutant #40 transformants obtained by direct selection on plates with acetamide are called AWC. Mutant #40 co-transformants growing well on acetamide are called AWCM.

The following number of (co-)transformants were further analyzed:

| Number of transformants | | Number of co-transformants | |
|---|---|---|---|
| AWC4155* | 3 | AWCM4155 | 3 |
| AWC4157 | 7 | AWCM4157 | 1 |
| AWC4159 | 2 | AWCM4159 | 5 |
| AWC4161 | 2 | AWCM4161 AWCM4163 | 2 2 |

| | | | |
|---|---|---|---|
| * 4155 indicates the presence of plasmid pUR4155NOT in the mutant #40 strain. | | | |

### 2.6 ScFv production by Aspergillus transformants

Analysis of *Aspergillus niger* var. *awamori* mutant # 40 transformants containing ScFv-fragment encoding sequences after culturing in medium with maltodextrin as an inducer.
AWC and AWCM transformants were grown in minimal medium (0,05% MgSO₄, 0,6% NaNO₃, 0,05% KCl, 0,15% KH₂PO₄ and trace elements) with 5% maltodextrin (Sigma Dextrin Corn type I; D-2006). Media were sterilized for 30 min at 120°C. Fifty ml medium (shake flask 300 ml) were inoculated with 4 x 10⁵ spores/ml, followed by culturing in an air incubator (300 rpm) at 30°C for different periods. Medium samples were taken after 45 to 50 hours and analyzed by SDS-PAGE followed by Western blot analyses. Furthermore a quantitative functional test was carried out by performing a Pin-ELISA assay.

### 2.6.1 Medium of ScFv-LYS and ScFv-HCG transformants

### 2.6.1a Western blot analysis and Coomassie Brilliant Blue-stained gels

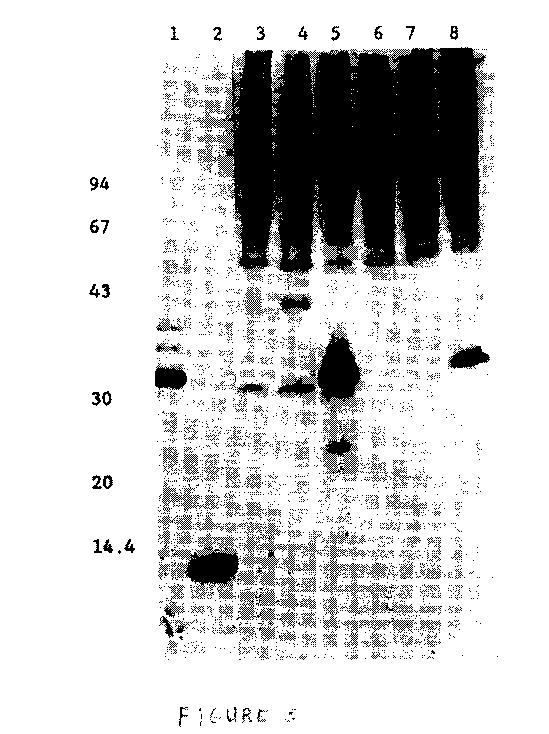

Western blot analysis of medium samples of AWC(M)4155 (18 a.a. glaA signal sequence-ScFv-LYS) (co-)transformants -in which anti-serum directed against Fv-LYS was used- revealed a band with a molecular mass of about 31 kDa which is absent in the medium of the mutant strain #40 (**Figure 5**). The presence of this band, which runs at the position of a protein with the expected size, points at secretion of ScFv-LYS in the culture medium.
In medium of several AWC(M)4159 (prepro-"glaA2"-KEX2-ScFv-LYS) (co-)transformants a similar, much stronger, band was found indicating a more efficient secretion of ScFv--LYS by these transformants. This protein band was also visible on Coomassie Brilliant Blue-stained gels.
In medium samples of AWC(M)4157 (18 aa. glaA signal sequence + ScFv-HCG) a faint band was found, while the band in medium of AWC(M)4161 (prepro-"glaA2"-KEX2-ScFv-HCG) (co-)transformants was clearly visible (molecular mass about 31 kDa). The aspecific signals were identical to the ones obtained with ScFv-LYS transformants. Some of the results are shown in **Figure 5** (Western blot).
Method: SDS-PAGE was carried out on 8-25% gradient gels using the Pharmacia Phast system or on homogeneous 12.5% home-made SDS-gels. For Western blot analysis a polyclonal anti-serum against Fv-LYS was used (1:1500) for the detection of both ScFv-LYS and ScFv-HCG.

### 2.6.1b Analysis by PIN-ELISA

The amount of functional ScFv-LYS (as determined by a PIN-ELISA assay) in the medium of AWC(M) transformants is given in **Table 2.**

**Table 2**

| Transformant: | construct | ScFv-fragment mg/l |
|---|---|---|
| AWCM4155 | #102 18 a.a. ss-glaA-ScFv-LYS | 15 - 22 -11 |
| AWCM4155 | #105 same | 3 |
| AWC 4155 | # 4 same | 10 |
| AWC 4155 | # 5 same | 2 |
| AWCM4159 | #101 prepro-"glaA2"-KEX2-ScFv-LYS | 91 - 66 - 67 |
| AWCM4159 | #608 same | 3 |
| AWCM4159 | #610 same | 16 |
| AWC 4159 | #701 same | 40 |
| AWCM4161 | #612 prepro-"glaA2"-KEX2-ScFv-HCG | 4 |
| AWC 4161 | # 2 same | 1 |
| *A. niger* var. *awamori* mutant #40 | | 0 |

The amount of ScFv-LYS in medium of AWC(M)4155 (18 a.a. glaA) transformants ranged from 2 to 22 mg/l. AWC(M)4159 (co-)transformants (prepro-"glaA2"-KEX2-construction) secrete up to about 90 mg/l into the medium, while no production was found for the *A. niger* var. *awamori* mutant #40 strain.
With the quantitative PIN-ELISA assay for the determination of ScFv-HCG it was found that AWC(M)4161 (co-)transformants ("glaA2"-KEX2-construction) secreted up to 4 mg/l functional ScFv-HCG into the medium. However, in the medium of AWC4157 (18 aa glaA signal sequence) transformants no ScFv-HCG was detected. Method: PINS coated with either lysozyme or HCG were incubated with (diluted) medium samples. Subsequently the PINs were incubated with antiserum against Fv-LYS and Fv-HCG respectively, then with goat-anti-rabbit conjugate with alkaline phosphatase. Finally the alkaline phosphatase enzyme-activity was determined after incubation with p-nitro-phenyl phosphate and the optical density was measured at 405 nm. Using standard solutions of Fv-LYS and Fv-HCG respectively, the amount of functional ScFv-LYS and ScFv-HCG was calculated.

### Example 3 Construction of Aspergillus niger var. awamori integration vectors for the production of ScFv fragments, using the endoxylanase promoter and terminator and a DNA sequence encoding the endoxylanase secretion signal and the mature endoxylanase protein.

Although this Example describes the construction of expression plasmids encoding fusion proteins between the mature endoxylanase protein and the ScFv fragment it is obvious that alternative expression plasmids can be constructed in much the same way in which only part of the endoxylanase protein is used.

### 3.1 Construction of pUR4158-A.

After digesting plasmid pScFvLYSmyc (see Example 1.1) with *Pst*I and *Xho*I*,* an about 0.7 kb *Pst*I*-Xho*I fragment could be isolated from agarose gel. This fragment codes for a truncated Single Chain Fv-Lys fragment missing the first 5 and the last 5 amino acids (see the nucleotide sequence (SEQ. ID. NO: 25) and deduced amino acid sequence (SEQ. ID. NO: 26) of the about 700 bp *Pst*I*-Xho*I fragment encoding the ScFv fragment of the monoclonal anti-lysozyme antibody D1.3 (ScFv LYS) given below.

The multiple cloning site of plasmid pEMBL9 (Dente *et al.*, 1983), ranging from the *Eco*RI to the *Hind*III site, can be replaced by a synthetic DNA fragment having the following nucleotide sequence (SEQ. ID. NO: 27-30).

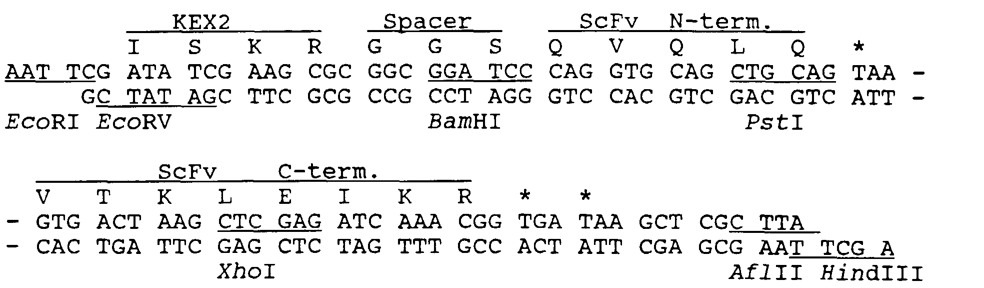

This DNA fragment can be used for replacing the multiple cloning site of plasmid pEMBL9 (ranging from the *Eco*RI to the *Hin*dIII site). The 5'-part of the coding strand of the synthetic DNA fragment codes for the KEX2 recognition site (ISKR), a spacer (GGS) followed by the first 5 amino acids of the variable part of the antibody heavy chain. The 3'-part of the coding sequence encodes the last 8 amino acid residues of the variable part of the antibody light chain. Upon digesting the obtained plasmid with *Pst*I and *Xho*I a vector fragment of about 4 kb can be isolated.
Upon ligating the about 0.7 kb *Pst*I*-Xho*I fragment of pScFvLYSmyc with the about 4 kb vector fragment, pUR4158-A can be obtained containing the restored genes encoding the V_{H} and V_{L} antibody fragments.

### 3.2 Construction of pXYL2.

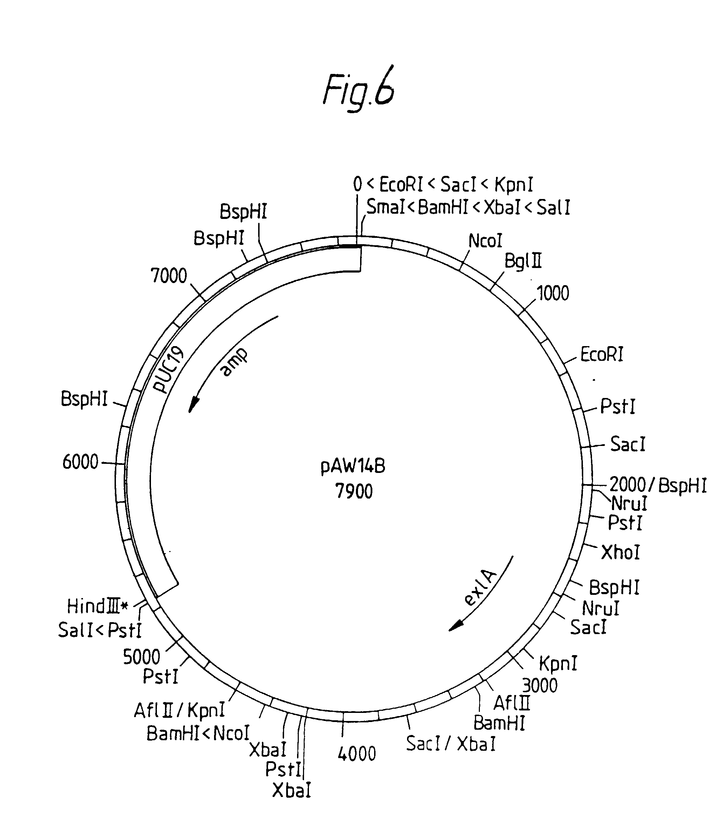

Plasmid pAW14B was the starting vector for the construction of a series of expression plasmids containing *exlA* expression signals and genes coding for ScFv fragments. The plasmid comprises an *Aspergillus niger* var. *awamori* chromosomal 5.2 kb *Sal*l fragment on which the 0.7 kb *exlA* gene is located, together with 2.5 kb of 5'-flanking sequences and 2.0 kb of 3'-flanking sequences (see **Figure 6** = Figure 3 of UNILEVER's not prior-published WO 93/12237). Upon digesting pAW14B with *Xba*I and *Bam*HI, an about 3.2 kb *Xha*I*-Bam*HI fragment can be isolated comprising the *exlA* promoter, the *exlA* structural gene and part of the *exlA* terminator area. This fragment can be cloned into plasmid pBluescript (ex Stratagene) digested with the same enzymes, resulting in plasmid pXYL1.
By applying PCR technology on the about 3.2 kb *Xba*I*-Bam*HI fragment, it is possible to change the 3'-end of the *exlA* structural gene by replacing the last codon encoding serine and the stop codon TAA by the *Bam*HI site GGA TCC followed by 8 other codons comprising an *Eco*RV site and an *Eco*RI site using a first (anti-sense) primer (**A**) given below (SEQ. ID. NO: 31-34) and a second (sense) primer (**B**) also given below located upstream of the *Sca*l site (located in the *exlA* gene). This sense primer corresponds with nucleotides 824-843 of Figure 1 of UNILEVER's not prior-published W) 93/12237 forming part of the *exlA* gene. After digesting the resulting PCR product with *Sca*I and *Eco*RI, an about 175 bp *Sca*I*-ECO*RI fragment can be isolated. Upon digesting pXYL1 with *Sca*I (partially) and with *Eco*RI (partially), an about 6 kb *Sca*I-*Eco*RI fragment, comprising the intact pBluescript DNA and the *exlA* promoter region and most of the *exlA* structural gene, can be isolated.
Ligation of the about 175 bp *Sca*I-*Eco*RI fragment with the about 6 kb *Sca*I-*Eco*RI fragment ex pXYL1 will result in a plasmid, called pXYL2, which differs from pXYL1 in that the 3'-part of the *exlA* gene and the terminator fragment are replaced by the newly obtained *Sca*I-*Eco*RI PCR fragment.

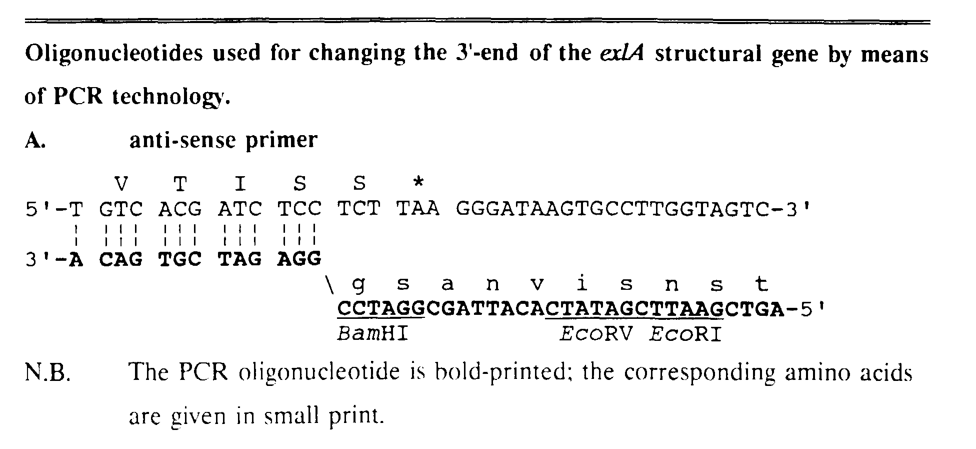

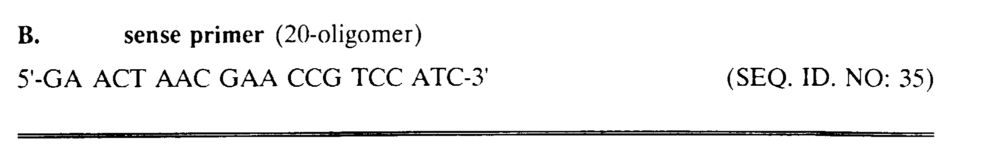

### 3.3 Construction of pUR4455 and pUR4456

Starting from pAW14B, pAW14B-10 was constructed by removing the *Eco*RI site originating from the pUC19 polylinker and introducing a *Not*I site.
This was achieved by partially digesting plasmid pAW14B with *Eco*RI and after dephosphorylation the linear 7.9 kb *Eco*RI plasmids were isolated and religated in the presence of the *"Eco*RI*"-Not*I linker:

After selecting a plasmid still containing the *Eco*RI site in the upstream area of the *exlA* structural gene, pAW14B-10 was obtained. Such selection method is known to a skilled person.
Subsequently the *Afl*II site, located downstream of the *exlA* terminator was removed by partially cleaving plasmid pAW14B-10 with *Afl*II and religating the isolated, linearized plasmid after filling in the sticky ends, resulting in plasmid pAW14B-11 after selecting the plasmid still containing the *Afl*II site near the stop codon of the *exlA* gene. Such selection method is known to a skilled person. This plasmid pAW14B-11 can be used for construction of a series of expression plasmids comprising a DNA fragment coding for a fusion protein consisting of the endoxylanase protein or part thereof and the ScFv fragment. Preferably the two protein fragments are connected by a protease recognition site e.g the KEX2 cleavage site.
(i) Upon digesting plasmid pAW14B-11 with *Not*I and *Afl*II, an about 4.7 kb fragment can be isolated comprising the pUC19 vector and part of the *exlA* terminator.
(ii) Upon digestion of pXYL2 with *Not*I and *Eco*RV, an about 3.2 kb fragment can be isolated. Alternatively an *Not*I-*Bam*HI fragment of about the same length can be isolated.
(iii) Upon digesting pUR4158-A with *Eco*RV and *Afl*II, an about 0.8 kb fragment can be isolated encoding the ScFv-LYS preceded by a short (linker) peptide comprising the KEX2 cleavage site and a spacer (GGS). Alternatively, a *Bam*HI-*Afl*II fragment of about the same length can be isolated, which fragment does not contain a DNA fragment encoding the KEX2 cleaving site.

A) For the construction of expression plasmids encoding the fusion protein consisting of mature endoxylanase and ScFv-LYS, the about 4.7 kb *Not*I*-Afl*II of pAW14B-11, the about 3.2 kb *Not*I*-Bam*HI fragment of pXYL2 and the about 0.75 kb *Bam*HI*-Afl*II fragment of pUR4158-A are ligated resulting in pUR4455.
B) For the construction of expression plasmids encoding the fusion protein consisting of mature endoxylanase and ScFv-LYS connected by the KEX2 cleavage site, the about 4.7 kb *Not*I-*Afl*II of pAW14B-11, the about 3.2 kb *Not*I*-Eco*RV fragment of pXYL2 and the about 0.75 kb *Eco*RV*-Afl*II fragment of pUR4158-A are ligated resulting in pUR4456.

The constructed expression vectors can subsequently be transferred to moulds (for example *Aspergillus niger, Aspergillus niger* var. *awamori, Aspergillus nidulans* etc.) by means of conventional co-transformation techniques and the chimeric gene comprising a DNA sequence encoding the desired ScFv fragment can then be expressed via induction of the endoxylanase II promoter. The constructed vector can also be provided with conventional selection markers (e.g. amdS or pyrG, hygromycin etc.), e.g. by introducing the corresponding genes into the unique *Not*I restriction site, and the mould can be transformed with the resulting vector to produce the desired protein, essentially as described in Example 2 of UNILEVER's not prior-published WO 93/12237.

### Example 4 Isolation of gene fragments of antibodies raised against (oral) microorganisms.

Monoclonal antibodies raised against oral microorganisms have been described in the literature (De Soet e*t al.*; 1990), an example of which is OMVU10 raised against streptococci. For the production of ScFv fragments derived from these monoclonal antibodies the gene fragments encoding the variable regions of the heavy and light chains had to be isolated. The isolation of RNA from the hybridoma cell lines, the preparation of cDNA and amplification of gene fragments encoding the variable regions of antibodies by PCR were performed according to standard procedures known from the literature (see for example Orlandi *et al,* 1989). For the PCR amplification different oligonucleotide primers have been used,
for the heavy chain fragment: in which S is C or G, M is A or C, R is A or G, and W is A or T and and for the light chain fragment (Kappa): and The heavy chain PCR fragment obtained in this way was digested with *PstI* and *Bst*EII and a *Pst*I-*Bst*EII fragment of about 0.33 kb was isolated. The thus obtained fragment can be cloned into pUR4158-A, To this end pUR4158-A is digested with *Pst*I and *Bst*EII, after which an about 4.4 kb vector fragment can be isolated. Ligation of the above described heavy chain fragment of OMVU10 with the about 4.4 kb vector fragment will result in pUR4158-A10H. In this plasmid the heavy chain fragment of the lysozym antibody, which was originally present, is replaced by that of the OMVU10 antibody.
The light chain PCR fragment obtained in a similar way was digested with *Sac*I and *Xho*I*,* and a *Sac*I*-Xho*I fragment of about 0.3 kb was isolated. After digestion of pUR4158-A10H with *Sac*I and *Xho*I*,* a vector fragment of about 4.4 kb can be isolated. Ligation of this vector fragment with the above described light chain fragment of OMVU10 will result in pUR4457. In this plasmid both the heavy chain fragment and the light chain fragment of the lysozyme antibody are replaced by the appropriate heavy and light chain fragments of OMVU10. The nucleotide sequence (SEQ. ID. NO: 41) and the deduced amino acid sequence (SEQ. ID. NO: 42) of the *Pst*I*-Xho*I fragment present in pUR4457 containing the thus obtained gene encoding an ScFv fragment of OMVU10 is given below. The first 5 codons and the last 5 codons are given in Example 3.1 above showing the overlap with the *Pst*I and *Xho*I sites.

### Example 5 Construction of an expression cassette for the production of an OMVU10 ScFv fragment.

After digesting pUR4457 (see Example 4) with *Eco*RV and *Afl*II, an about 0.8 kb fragment can be isolated encoding the ScFv-OMVU10 preceded by a short (linker) peptide comprising the KEX2 cleavage site and the GGS spacer. Alternatively, a *Bam*HI-*Afl*II fragment of about 0.75 kb can be isolated for the construction of expression plasmids coding for fusion proteins not containing a KEX2 cleavage site.
Upon ligating the thus obtained fragments with the fragments obtained in 3.3 (i) and (ii) in the same way as described in 3.3 B) and A), an expression plasmid can be obtained containing a DNA sequence coding for a fusion protein comprising the endoxylanase protein and the ScFv OMVU10 fragment, either with (pUR4460) or without (pUR4459) the KEX2 cleavage site, respectively.
Analogous to the method described in Example 3, the resulting plasmids (either with or without an added selection marker) can be introduced into *Aspergillus.*

### Example 6 Isolation of gene fragments of an antibody raised against human pregnancy hormone (HCG).

In much the same way as described in Example 4, gene fragments coding for the variable regions of the heavy and the light chains of anti-HCG antibodies were isolated and can be cloned into plasmid pUR4158-A which results in plasmid pUR4458. The nucleotide sequence (SEQ. ID. NO: 7) and the deduced amino acid sequence (SEQ. ID. NO: 8) of the *Pst*I*-Xho*I fragment encoding the ScFv-HCG fragment were given above in Example 1.2.

### Example 7 Construction of expression cassettes for the production of ScFv fragments, using the endoxylanase promoter and terminator and a DNA sequence encoding the prepro-"glaA2" protein.

### 7.1 Construction of pAW14B-12.

Plasmid pAW14B-12 was constructed using pAW14B-11 (see Example 3.3) as starting material. After digestion of pAW14B-11 with *Afl*II (located at the *exlA* stop codon) and *Bgl*II (located in the *exlA* promoter) the 2.4 kb *Afl*II*-Bgl*II fragment, containing part of the *exlA* promoter and the *exlA* gene was isolated.
After partial digestion of this fragment with *Bsp*HI (located in the *exlA* promoter and the *exlA* start codon) the isolated 1.8 kb *Bgl*II*-Bsp*HI *exlA* promoter fragment (up to the ATG) was ligated with the isolated 5.5 kb *Afl*II-*Bgl*II fragment of pAW14B-11, containing the *exlA* terminator, in the presence of the synthetic DNA oligonucleotides: resulting in pAW14B-12.

### 7.2 Assembly of expression cassettes

(i) Upon digesting pAW14B-12 with *Bbs*I (partially) and *Afl*II, an about 7.3 kb *Bsp*HI-*Afl*II vector fragment was isolated.
(ii) From plasmid pAN56-4 (described in the above mentioned reference of M.P. Broekhuijsen *et al.)* an about 1.9 kb *Nco*I*-Eco*RV fragment was isolated, comprising part of the *glaA* gene, starting from the ATG initiation codon (which coincides with the *Nco*I site), and coding for the glucoamylase prepro part and the first 514 amino acids of the mature glucoamylase ("glaA2").
(iii) From the plasmids pUR4158-A (encoding for the ScFv-LYS fragment preceded by the KEX2 recognition site and the GGS spacer: see Example 3.1), pUR4457 (encoding for the ScFv-OMVU10 fragment preceded by the KEX2 recognition site and the GGS spacer: see Example 4), and pUR4458 (encoding for the ScFv-HCG fragment preceded by the KEX2 recognition site and the GGS spacer: see Example 6) *Eco*RV-*Afl*II fragments of about 0.8 kb were isolated.

Upon ligating (i) the *BSp*HI*-Afl*II vector fragment, (ii) the *Nco*I*-Eco*RV *glaA* fragment (*Nco*I sticky ends are compatible with BspHI sticky ends), and either of the *Eco*RV-*Afl*II ScFv encoding fragments, a set of expression plasmids can be obtained.
pUR4462 *PexlA -* prepro-"glaA2"-KEX2-ScFv-LYS
pUR4463 *PexlA -* prepro-"glaA2"-KEX2-ScFv-HCG
pUR4464 *PexlA* - prepro-"glaA2"-KEX2-ScFv-OMVU10

After insertion of the *amd*S selection marker into the *Not*I site, the resulting plasmids were introduced into *Aspergillus,* as described in Example 3.

### 7.3 Production of ScFv-LYS

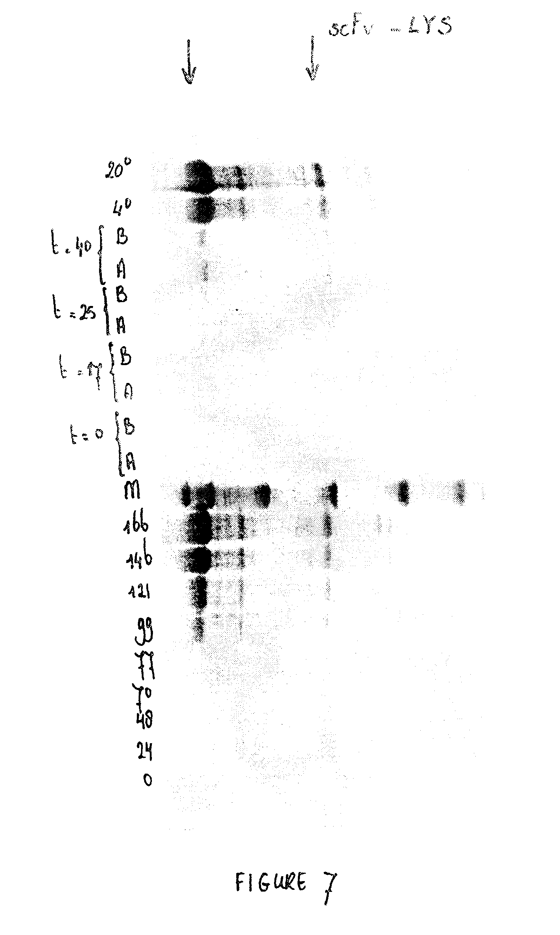

Upon growth of the resulting *Aspergillus niger* var. *awamori* transformed with pUR4462 in a 10 litre fermenter, the culture medium was analyzed by polyacrylamide gel electrophoresis. **Figure 7** shows the gel after it was stained with Coomassie Brilliant Blue and with arrows are indicated the released ScFv-LYS fragment and the fusion protein and/or the truncated glaA protein.
The amount of "active" ScFv-LYS was determined to be about 250 mg/l. It is obvious that further optimization of the fermentation conditions or mutagenesis of the production strain will result in even higher production levels.

### References

- **WO 90/15860** (GENENCOR INC. / V.B. Lawlis; published 27 Dec. 1990; DNA sequences, vectors, and fusion polypeptides to increase secretion of desired polypeptides from filamentous fungi)
- **WO 91/19782** (UNILEVER / R.F.M. Van Gorcom, J.G.M. Hessing, J. Maat, M. Roza & J.M.A. Verbakel; published 26 Dec. 1991; Xylanase production)
- **WO 92/01797** (OY ALKO AB / E. Nyyssönen, S. Keränen, M. Penttilä, K. Takkinen & J.K.C. Knowles; published 6 Feb. 1992; Immunoglobulin production by *Trichodema*)
- **WO 93/02198** (TECH. RES. INST. FINLAND / T.T. Teeri, K. Takkinen, M-L. Laukkanen, K. Alfthan, D. Sizmann, J.K. Knowles; published 4 Feb. 1993; Recombinant secretable fusion proteins)
- **not prior-published PCT application PCT/EP92/02896**, filed 9 December 1992 and claiming a priority date of 9 December 1991, published during the priority year as **WO 93/12237** on 24 June 1993 (UNILEVER / R.J. Gouka, C.A.M.J.J. Van den Hondel, W. Musters, H. Stam & J.M.A. Verbakel; Process for producing/secreting a protein by a transformed mould using expression/secretion regulating regions derived from an *Aspergillus* endoxylanase II gene
- **Broekhuijsen, M.P.,** Mattern, I.E., Contreras, R., Kinghorn, J.R., and Van den Hondel, C.A.M.J.J.; J. Biotechnology 31, No.2 (1993) 135-145; Secretion of heterologous proteins by *Aspergillus niger:* Production of active human interleukin-6 in a protease-deficient mutant by KEX2-like processing of a glucoamylase-hIL6 fusion protein
- **Calmels, T.P.G.,** Martin, F., Durand, H., and Tiraby, G.; J. Biotechnol. **17** (1991) 51-66; Proteolytic events in the processing of secreted proteins in fungi
- **Contreras, R.**, Carrez, D., Kinghorn, J.R., Van den Hondel, C.A.M.J.J., and Fiers, W.; Bio/Technology **9** (1991) 378-381; Efficient KEX2-like processing of a glucoamylase-interleukin-6 fusion protein by *Aspergillus nidulans* and secretion of mature interleukin-6
- **De Soet,** J.J., Van Dalen, P.J., Russell, R.R.B., and De Graaff, J.; Antonie van Leeuwenhoek **58** (1990) 219-225; Identification of mutants streptococci with monoclonal antibodies
- **Dente, L.**, Cesareni, G. and Cortese, R.; Nucleic Acids Research **11** (1983) 1645-1655; pEMBL: a new family of single stranded plasmids
- **Fuller, R.S.**, Sterne, R.E., and Thorner J.; Ann. Rev. Physiol. **50** (1988) 345-362; Enzymes required for yeast prohormone processing
- **Hynes M.J.,** Corrick C.M., and King J.A.; Mol. Cell Biol. 3 (1983) 1430-1439; Isolation of genomic clones containing the *amd-s* gene of *Aspergillus nidulans* and their use in the analysis of structural and regulatory mutations
- **Jones, P.T.; Dear P.H.; Foote J.; Neuberger, M.S., and Winter, G.;** Nature 321 (1986) 522-525; Replacing the complementary determining regions in a human antibody with those from a mouse
- **Matthews, J.D.** and Wells, J.A.; Science **260** (21 May 1993) 1113-1117; Substrate Phage: Selection of Protease Substrates by Monovalent Phage Display
- **Nyyssönen, E.**, Penttilä, M., Harkki, A., Saloheimo, A., Knowles, J.K.C., and Keränen, S.; BIO/TECHNOLOGY 11 (May 1993) 591-595; Efficient Production of Antibody Fragments by the Filamentous Fungus *Trichoderma reesei*
- **Orlandi, R.,** Güssow, D.H., Jones, P.T., and Winter, G.; Proc. Natl. Acad. Sci. USA **86** (1989) 3833-3837; Cloning immunoglobulin variable domains for expression by the polymerase chain reaction
- **Punt, PJ.,** Zegers, N.D., Busscher, M., Pouwels, P.H. and Van den Hondel, C.A.M.J.J.; J. Biotech 17 (1991) 19-34; Intracellular and extracellular production of proteins in *Aspergillus* under the control of expression signals of the highly expressed *Aspergillus nidulans gpd-a* gene
- **Punt, P.J.** and Van den Hondel, C.A.M.J.J.; Transformation of filamentous fungi based on hygromycin B and phleomycin resistance markers. In: *Methods in Enzymology* Vol. 216. Eds: J.N. Abelson and M.I. Simon. Academic Press, Inc., Orlando, Florida, (1992) pp. 447-457
- **Sambrook, J.,** Fritsch, E.F. & Maniatis, T. *Molecular Cloning: A Laboratory Manual* 2nd Edn (Cold Spring Harbor Laboratory Press, New York, 1989).
- **Van den Hondel, C.A.M.J.J.**, Punt, P.J., and Van Gorcom, R.F.M.; Heterologous gene expression in filamentous fungi. In: More gene manipulation in fungi (Eds. J.W. Bennett amd L.L. Lasure), Academic Press, (1991) pp 396-428
- **Ward, M.,** Wilson, L.J., Kodama, K.H., Rey, M.W., and Berka, R.M.; Bio/Technology **8** (May 1990) 435-440; Improved production of chymosin in Aspergillus by expression as a glucoamylase-chymosin fusion
- **Ward, S.,** Güssow, D., Griffiths, A.D., Jones, P.T., and Winter, G.; Nature **341** (1989) 544-546; Binding activities of a repertoire of single immunoglobulin variable domains secreted from *E. coli*
- **Wernars, K.;** "DNA mediated transformation of the filamentous fungus *Aspergillus nidulans";* thesis (1986); Landbouw Hogeschool Wageningen.

## Claims

1. A process for producing fusion proteins comprising ScFv fragments by a transformed mould, in which
(a) the mould belongs to the genus *Aspergillus,* and
(b) the *Aspergillus* contains a DNA sequence encoding the ScFv fragment under control of at least one expression and/or secretion regulating region derived from a mould selected from the group consisting of promoter sequences, terminator sequences and signal sequence-encoding DNA sequences, and functional derivatives or analogues thereof, **optionally** followed by a proteolytic cleavage step for separating the ScFv fragment part from the fusion protein.

2. A process according to claim 1, in which said "at least one expression and/or secretion regulating region derived from a mould" is the combination of both a promoter sequence and signal sequence-encoding DNA sequence derived from a glucoamylase gene ex *Aspergillus* plus a terminator sequence of a *trpC* gene ex *Aspergillus.*

3. A process according to claim 1, in which said "at least one expression and/or secretion regulating region derived from a mould" is derived from endoxylanase II gene (*exlA* gene) of *Aspergillus niger* var. *awamori* present on plasmid pAW14B, which is present in a transformed E.coli strain JM 109 (deposit No CBS 237.90).

4. A process according to claim 1, in which said DNA sequence encoding the ScFv fragment forms part of a chimeric gene encoding a fusion protein, whereby said DNA sequence encoding the ScFv fragment is preceded at its 5' end by at least part of a structural gene encoding the mature part of a secreted mould protein.

5. A process according to claims 4, in which said structural gene encodes an endoxylanase or a glucoamylase.

6. A process according to claim 4, in which said ScFv fragment in the fusion protein is bound to said secreted mould protein or part thereof by a proteolytic cleavage site.

7. A process according to claim 6, in which said cleavage site is a KEX2-like site.

8. A process according to any one of claims 1-7, in which the mould is cultured under such conditions that the yield of ScFv fragment is at least 40 mg/l, preferably at least 60 mg/l, more preferably at least 90 mg/l and still more preferably at least 150 mg/l.

9. A process for producing fusion proteins comprising ScFv fragments by a transformed mould, in which
(a) the mould belongs to one of the genera *Mucor, Neurospora* and *Penicillium,* and
(b) the mould contains a DNA sequence encoding the ScFv fragment under control of at least one expression and/or secretion regulating region derived from a mould selected from the group consisting of promoter sequences, terminator sequences and signal sequence-encoding DNA sequences, and functional derivatives or analogues thereof, **optionally** followed by a proteolytic cleavage step for separating the ScFv fragment part from the fusion protein, whereby **optionally** the mould is cultured under such conditions that the yield of ScFv fragment is at least 40 mg/l, preferably at least 60 mg/l, more preferably at least 90 mg/l and still more preferably at least 150 mg/l.

## Patentansprüche

1. Verfahren zur Herstellung von Fusionsproteinen, die ScFv-Fragmente enthalten, mit transformiertem Schimmel, bei dem
a) der Schimmel zur Gattung der *Aspergillus* gehört, und
b) der *Aspergillus* eine DNS-Sequenz enthält, die für das ScFv-Fragment kodiert unter der Steuerung von mindestens einer eine Expression und/oder eine Sekretion regulierenden, von einem Schimmel abstammenden Region, die ausgewählt ist aus der Gruppe von Promotorsequenzen, Stoppsequenzen und für Signalsequenzen kodierenden DNS-Sequenzen sowie funktionalen Derivaten oder Analogen hiervon,
gegebenenfalls gefolgt von einem proteolytischen Spaltungsschritt zur Abtrennung des ScFv-Fragmentteils vom Fusionsprotein.

2. Verfahren nach Anspruch 1, bei dem die "mindestens eine Expression und/oder eine Sekretion regulierende, von einem Schimmel abstammende Region" die Kombination aus einer Promotorsequenz und einer für eine Signalsequenz kodierenden DNS-Sequenz ist, die von einem Glucoamylasegen ex *Aspergillus* und einer Stoppsequenz eines trpC Gens ex *Aspergillus* ist.

3. Verfahren nach Anspruch 1, bei dem die "mindestens eine Expression und/oder eine Sekretion regulierende, von einem Schimmel abstammende Region" von einem Endoxylanase II Gen (exlA genc) des As*pergillus niger var. awamori,* das sich auf dem Plasmid pAW14B befindet, das in einer transformierten E.coli-Kette JM 109 (Hinterlegungsnr. CBS 237.90) vorliegt, abstammt.

4. Verfahren nach Anspruch 1, bei dem das für eine DNS-Sequenz kodierende ScFv-Fragment ein Teil eines heterozygoten Gens ist, das für ein Fusionsprotein kodiert, wobei der DNS-Sequenz an seinem 5'-Ende mindestens ein Teil eines Strukturgens voransteht, das "für den reifen Teil eines abgesonderten Schimmelproteins kodiert.

5. Verfahren nach Anspruch 4, bei dem das Strukturgen für eine Endoxylanase oder eine Glucoamylase kodiert.

6. Verfahren nach Anspruch 4, bei dem das ScFv-Fragment Im Fusionsprotein an das abgesonderte Schimmelprotein oder einen Teil hiervon über eine proteolytische Spaltort gebunden ist.

7. Verfahren nach Anspruch 6, bei dem der Spaltort ein KEX2-artiger Ort ist.

8. Verfahren nach einem der vorstehenden Ansprüche 1 bis 7, bei dem der Schimmel unter solchen Bedingungen kultiviert wird, daß der Ertrag an ScFv-Fragmenten mindestens 40 mg/l, vorzugsweise mindestens 60 mg/l, bevorzugter mindestens 90 mg/l und noch bevorzugter mindestens 150 mg/l beträgt.

9. Verfahren zur Herstellung von Fusionsproteinen, die ScFv-Fragmente enthalten, mit transformiertem Schimmel, bei dem
a) der Schimmel zu einer der Gattungen *Mucor, Neurospora* und *Penicillium* gehört, und
b) der *Aspergillus* eine DNS-Sequenz enthält, die für das ScFv-Fragment kodiert unter der Steuerung von mindestens einer eine Expresslon und/oder eine Sekretion regulierenden, von einem Schimmel abstammenden Region, die ausgewählt ist aus der Gruppe von Promotorsequenzen, Stoppsequenzen und für Signalsequenzen kodierenden DNS-Sequenzen sowie funktionalen Derivaten oder Analogen hiervon,
gegebenenfalls gefolgt von einem proteolytischen Spaltungsschritt zur Abtrennung des ScFv-Fragmentteils vom Fusionsprotein, wobei der Schimmel gegebenenfalls unter solchen Bedingungen kultiviert wird, daß der Ertrag an ScFv-Fragmenten mindestens 40 mg/l, vorzugsweise mindestens 60 mg/l, bevorzugter mindestens 90 mg/l und noch bevorzugter mindestens 150 mg/l beträgt.

## Revendications

1. Procédé de production de protéines de fusion contenant des fragments ScFv à l'aide d'une moisissure transformée, caractérisé en ce que
(a) la moisissure appartient au genre *Aspergillus,* et
(b) *l'Aspergillus* contient une séquence d'ADN codant pour le fragment ScFv sous le contrôle d'au moins une région de régulation de l'expression et/ou de la sécrétion dérivée d'une moisissure choisie dans un groupe constitué de séquences promoteur, de séquences de terminaison et de séquences d'ADN codant pour une séquence de signal, et les dérivés et analogues fonctionnels de celles-ci,
éventuellement suivi par une étape de clivage protéolytique pour séparer la partie du fragment ScFv de la protéine de fusion.

2. Procédé selon la revendication 1, caractérisé en ce que ladite "au moins une région de régulation de l'expression et/ou de la sécrétion dérivée d'une moisissure" est l'association d'une séquence promoteur et d'une séquence d'ADN codant pour une séquence de signal dérivées du gêne de la glucoamylase ex. *Aspergillus* plus la séquence de terminaison du gêne de trpC ex. *Aspergillus.*

3. Procédé selon la revendication 1, caractérisé en ce que ladite "au moins une région de régulation de l'expression et/ou de la sécrétion dérivée d'une moisissure" est dérivée du gène de l'endoxylanase II (gène exlA) *d'Aspergillus niger* var. *awamori* présent sur le plasmide pAW14B, qui est présent dans une souche transformée de *E. coli* JM 109 (dépôt n° CBS 237.90).

4. Procédé selon la revendication 1, caractérisé en ce que ladite séquence d'ADN codant pour le fragment ScFv fait partie d'un gène chimérique codant pour une protéine de fusion, par lequel ladite séquence d'ADN codant pour le fragment ScFv est précédé à sa terminaison S par au moins une partie d'un gène structurel codant pour la partie mature d'une protéine sécrétée de la moisissure.

5. Procédé selon la revendication 4, caractérisé en ce que ledit gène structurel code pour une endoxylanase ou une glucoamylase.

6. Procédé selon la revendication 4, caractérisé en ce que ledit fragment ScFv dans la protéine de fusion est lié à ladite protéine sécrétée de la moisissure ou à une partie de celle-ci par un site de clivage protéolytique.

7. Procédé selon la revendication 6, caractérisé en ce que ledit site de clivage est un site de type KEX2.

8. Procédé selon l'une quelconque des revendications 1-7, caractérisé en ce que la moisissure est cultivée dans des conditions telles que le rendement en fragment ScFv est au moins de 40 mg/l, de préférence au moins 60 mg/l, plus préférentiellement au moins 90 mg/l et encore plus préférentiellement au moins 150 mg/l.

9. Procédé de production de protéines de fusion contenant des fragments ScFv à l'aide d'une moisissure transformée, caractérisé en ce que
(a) la moisissure appartient à l'un des genres *Mucor, Neurospora* et *Penicillium*, et
(b) la moisissure contient une séquence d'ADN codant pour le fragment ScFv sous le contrôle d'au moins une région de régulation de l'expression et/ou de la sécrétion dérivée d'une moisissure choisie dans un groupe constitué de séquences promoteur, de séquences de terminaison et de séquences d'ADN codant pour une séquence de signal, et les dérivés et analogues fonctionnels de celles-ci,
éventuellement suivi par une étape de clivage protéolytique pour séparer la partie du fragment ScFv de la protéine de fusion, caractérisé en ce que éventuellement la moisissure est cultivée dans des conditions telles que le rendement en fragment ScFv est au moins de 40 mg/l, de préférence au moins 60 mg/l, plus préférentiellement au moins 90 mg/l et encore plus préférentiellement au moins 150 mg/l.
